(19) Europäisches Patentamt
European Patent Office
Office européen des brevets

(11) EP 2 347 751 A1

(12) **DEMANDE DE BREVET EUROPEEN**

(43) Date de publication:
**27.07.2011 Bulletin 2011/30**

(21) Numéro de dépôt: **11150357.9**

(22) Date de dépôt: **07.01.2011**

(51) Int Cl.:
*A61K 8/02* (2006.01) *A61K 8/37* (2006.01)
*A61K 8/85* (2006.01) *A61K 8/92* (2006.01)
*A61Q 1/06* (2006.01) *A45D 40/08* (2006.01)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **22.01.2010 FR 1050442**

(71) Demandeur: **L'Oréal**
**75008 Paris (FR)**

(72) Inventeurs:
- **Ilekti, Philippe**
**94700, Maison-Alfort (FR)**
- **Schwartz, Véronique**
**92290, Chatenay Malabry (FR)**

(74) Mandataire: **Kromer, Christophe**
**L'Oréal**
**D.I.P.I.**
**25-29 Quai Aulagnier**
**92665 Asnières-sur-Seine (FR)**

(54) **Procédé de traitement cosmétique chauffant mettant en oeuvre un polyester particulier**

(57) La présente invention concerne un procédé de maquillage et/ou de soin non thérapeutique des matières kératiniques humaines non fibreuses, notamment la peau, ses muqueuses ou les ongles, comportant les étapes consistant à :
- amener au contact ou au voisinage d'un dispositif de chauffage une surface extérieure d'un morceau de composition cosmétique solide de manière à chauffer ledit morceau de façon localisée en vue de ne ramollir essentiellement que ladite surface extérieure et à en abaisser le coefficient de frottement dynamique et
- appliquer ensuite la surface extérieure de la composition ainsi réchauffée sur la région à traiter,

ladite composition cosmétique solide comprenant dans un milieu physiologiquement acceptable :
(i) au moins un polyester susceptible d'être obtenu par réaction :
- d'un tétraol ayant de 4 à 10 atomes de carbone ;
- d'un acide saturé monocarboxylique, linéaire ou ramifié, ayant de 9 à 23 atomes de carbone ;
- d'un diacide carboxylique cyclique ayant de 6 à 12 atomes de carbone ; et
- d'un acide monocarboxylique aromatique ayant de 7 à 11 atomes de carbone,

(ii) et au moins un corps gras solide, choisi parmi les cires et les corps gras pâteux, et leur mélange.

EP 2 347 751 A1

## Description

La présente invention concerne le domaine du soin et/ou du maquillage des matières kératiniques humaines et plus particulièrement de la peau et/ou des lèvres et en particulier un procédé de maquillage comprenant une étape de chauffage préalable à l'application d'une composition comprenant au moins un polyester particulier et un corps gras solide.

La mise au point de formulations, en particulier sous forme solide, dédiées au maquillage et/ou au soin de la peau et/ou des lèvres, dotées de propriétés satisfaisantes en termes d'application, notamment de glissant et de quantité déposée et dont le dépôt sur les matières kératiniques (en particulier la peau et/ou les lèvres) soit satisfaisant en termes de brillance, de confort et de tenue de la couleur, relève d'un objectif permanent.

Il est connu du document FR 2 917 616, des compositions cosmétiques contenant des polyesters associé à des huiles volatiles classiques telles que l'isododécane, pour obtenir après application sur les matières kératiniques, notamment les lèvres, un dépôt de maquillage relativement brillant et présentant des propriétés de tenue acceptable.

Cependant, il est toujours souhaitable d'améliorer encore la tenue du dépôt de telles compositions lorsqu'elles sont appliquées sur les matières kératiniques, en particulier de façon à ce que le dépôt formé conserve son intégrité et reste homogène pendant 2 ou 3 heures, voire pendant 4 ou 6 heures, et n'ait pas tendance à se fragiliser ou se fragmenter, en particulier au contact des matières grasses, en particulier lors d'un repas, ou au contact de la salive. De plus, dans le cas de compositions de rouge à lèvres il est souhaitable d'améliorer la brillance du dépôt de telles compositions sur les lèvres.

De plus, les polyesters sont très visqueux, et les compositions solides résultantes sont généralement très accrocheuses au contact de la peau ou des lèvres, et donc difficiles à appliquer, ne permettant pas d'obtenir un dépôt épais et homogène. Elles glissent mal et se délitent difficilement.

On cherche donc à obtenir des compositions solides (en particulier compatibles avec le conditionnement en bâtonnet ou sous une autre forme solide) comprenant de tels polyesters, qui soient stables, qui s'appliquent facilement (bon glissement) sur la peau ou les lèvres, et formant un dépôt présentant des bonnes propriétés de tenue dans le temps, et éventuellement de brillance dans le cas de compositions de rouge à lèvres, et qui soit confortable.

L'invention a précisément pour objet, de proposer un nouveau mode de maquillage et/ou de soin permettant de donner satisfaction à l'ensemble des exigences précitées.

Ainsi, selon un premier de ses aspects, un procédé de maquillage et/ou de soin non thérapeutique des matières kératiniques humaines notamment la peau ou les lèvres, dans lequel

- on amène au contact ou au voisinage d'un dispositif de chauffage une surface extérieure d'un morceau de composition cosmétique solide à 20°C de manière à chauffer ledit morceau de façon localisée en vue de ne ramollir essentiellement que ladite surface extérieure et à en abaisser le coefficient de frottement dynamique, et
- on applique ensuite la surface extérieure de la composition ainsi réchauffée sur la région à traiter, et notamment à maquiller,
  ladite composition cosmétique solide comprenant dans un milieu physiologiquement acceptable :

  (i) au moins un polyester susceptible d'être obtenu par réaction :

  - d'un tétraol ayant de 4 à 10 atomes de carbone ;
  - d'un acide saturé monocarboxylique, linéaire ou ramifié, ayant de 9 à 23 atomes de carbone ;
  - d'un diacide carboxylique cyclique ayant de 6 à 12 atomes de carbone ; et
  - d'un acide monocarboxylique aromatique ayant de 7 à 11 atomes de carbone,

  (ii) et au moins un corps gras solide choisi parmi les cires et les corps gras pâteux, et leur mélange

Selon un mode de réalisation particulier de l'invention, le procédé est un procédé de maquillage.

Selon un mode de réalisation particulier, la surface extérieure ramollie est portée au contact direct de la région à traiter, et en particulier des matières kératiniques.

Autrement dit, aucun applicateur n'est employé pour déposer la composition ramollie.

Au sens de la présente invention, on entend par « solide » une composition présentant, à 20°C et à pression atmosphérique (760 mm de Hg), une dureté supérieure à 30 $Nm^{-1}$, dé préférence supérieure à $40Nm^{-1}$.

Selon un mode de réalisation préféré, la composition solide selon l'invention est sous forme d'un stick. Lorsque la composition est sous forme d'un stick, la surface extérieure peut être définie comme l'extrémité de celui-ci, c'est-à-dire l'extrémité libre qui est appliquée sur les matières kératiniques par l'utilisateur.

Selon un mode de réalisation particulier, le procédé selon la présente invention est tel que la composition est sous forme de bâtonnet, notamment de diamètre supérieur ou égal à 7mm.

Selon encore un autre mode de réalisation, le procédé selon la présente invention est tel que la composition est un rouge à lèvres.

Elle est avantageusement caractérisée par une dureté telle que définie ci-après.

Selon encore un autre aspect, l'invention concerne un kit comportant :

- une composition telle que définie précédemment, et
- un dispositif de chauffage permettant de chauffer localement une surface d'un morceau de ladite composition.

Le morceau de composition peut être en permanence au contact ou à proximité du dispositif de chauffage et celui-ci peut être activé préalablement à l'application de la composition, pour élever la température de la surface extérieure du morceau de composition. En variante, le morceau de composition n'est amené au contact ou à proximité du dispositif de chauffage que pour l'utilisation, en vue de l'application de la composition

Ainsi, l'invention peut permettre de chauffer en surface, juste avant l'application, par exemple le haut du biseau d'un bâtonnet de rouge à lèvres réalisé avec une composition selon l'invention, afin de permettre le dépôt, et ceci même si le bâtonnet contient des composés peu propices à une application satisfaisante à froid, ces composés apportant des performances accrues en termes de tenue et/ou de brillance.

Dans des exemples de mise en oeuvre de l'invention, en réchauffant la surface du bâtonnet, on peut améliorer son glissement et donc son application sur les lèvres ou la peau.

Selon un mode de réalisation particulier, la composition mise en oeuvre selon l'invention présente un coefficient de frottement dynamique sensible à la température, supérieur ou égal à 0,5 à 25 °C, mieux supérieur ou égal à 0,6 à 25 °C.

La composition solide présente avantageusement une dureté supérieure ou égale à 80 $Nm^{-1}$ à 20 °C, mieux supérieure ou égale à 100 $Nm^{-1}$, voire à 120 $Nm^{-1}$ à 20 °C, ce qui rend le bâtonnet résistant sur le plan mécanique et permet son conditionnement par exemple dans un étui conventionnel comportant deux parties pouvant tourner l'une par rapport à l'autre pour déplacer le bâtonnet.

Le coefficient de frottement dynamique peut être, à la température à laquelle la composition chauffée, est inférieur ou égal à 0,45, mieux à 0,4.

Le coefficient de frottement dynamique qui est supérieur ou égal à 0,5 à 25 °C peut ainsi devenir par exemple inférieur ou égal à 0,45 à 45 °C, c'est-à-dire atteindre une valeur comparable à certains rouges à lèvres connus prévus pour une application à 25 °C.

Le coefficient de frottement dynamique qui est supérieur ou égal à 0,5 à 25 °C peut ainsi devenir par exemple inférieur ou égal à 0,45 à 45 °C, c'est-à-dire atteindre une valeur comparable à certains rouges à lèvres connus prévus pour une application à 25 °C.

L'invention peut s'appliquer à un bâtonnet de produit comportant une quantité en polyester telle que son application à froid et/ou sans chauffage soit difficile, quasi-impossible ou désagréable. Pour un tel bâtonnet de produit, l'application après chauffage devient possible, avec des performances de confort voire de brillance au regard de la présence des huiles qu'il contient particulièrement avantageuses.

Dans le cadre de l'invention, il est important que les compositions mises en oeuvre soient stables (en particulier qui ne présentent pas de problème de déphasage) à température ambiante (à 20°C) et lorsqu'elles sont chauffées avant application (notamment à une température de l'ordre de 60°C).

Le produit peut être chauffé de diverses manières, par exemple en étant exposé à un rayonnement infrarouge ou à un rayonnement radioélectrique.

Le produit peut encore être chauffé par soufflage d'air chaud, en étant exposé à des vibrations ultrasonores ou par transfert de chaleur au contact ou à proximité d'une surface chaude, laquelle vient par exemple en appui radialement contre la surface extérieure, notamment l'extrémité du bâtonnet. La surface chaude peut aussi venir en appui axialement contre la surface extérieure, notamment l'extrémité du bâtonnet. La surface chaude peut avoir une forme de biseau, de cône inversé ou de creux concave, notamment sphérique.

La surface extérieure du produit peut être chauffée à une température $T_f$ supérieure ou égale à 40 °C, voire supérieure à 45 °C ou encore supérieure à 50 °C. La surface extérieure peut être chauffée à une température $T_f$ comprise entre 40 °C et 95 °C, mieux 45 °C à 85 °C, encore mieux 45 °C à 75 °C.

La température de la surface d'application, notamment de l'extrémité du bâtonnet, ne doit pas entraîner de risque de brûlure au moment de l'application. C'est pourquoi un temps d'attente entre le moment où l'extrémité est chauffée et l'application sur les matières kératiniques peut éventuellement être nécessaire.

L'écart de température entre la surface extérieure chauffée et la portion du produit qui reste solide peut être supérieur ou égal à 5 °C, mieux supérieur ou égal à 15 °C ou 20 °C, au moins au début de l'application, voire supérieure à 30 °C.

Seul le produit peut venir au contact de la région traitée lors de l'application.

Le dispositif de chauffage peut être logé dans un capot de fermeture du support, de manière à permettre de chauffer la surface extérieure avec le capot en place sur le support. Le dispositif de chauffage peut aussi être logé

ailleurs que dans un capot de fermeture du support.

Le dispositif de chauffage peut être logé dans un boîtier sur lequel peut être engagé le support de manière à ce que le chauffage puisse avoir lieu quand le support est engagé dans le boîtier, notamment un boîtier comportant une ouverture dans laquelle le morceau de produit solide peut être engagé, de préférence sans que l'ensemble du support ne soit disposé à l'intérieur du boîtier.

Le dispositif de chauffage peut être solidaire du dispositif de conditionnement et d'application.

Le dispositif de chauffage peut être agencé pour venir au contact de la surface extérieure.

Le dispositif de chauffage peut être agencé pour être traversé par le morceau de produit, notamment comporter une surface chaude de forme annulaire.

Le dispositif de chauffage peut comporter un moyen de commande permettant à l'utilisateur de commander son fonctionnement. Ce moyen de commande peut comporter un interrupteur présent sur le support ou sur un capot de fermeture du support.

Le dispositif de chauffage peut comporter une résistance électrique pour chauffer une surface pouvant venir au contact de la surface d'application ou à proximité de celle-ci.

Le dispositif de chauffage peut comporter un émetteur d'infrarouges agencé pour soumettre la surface d'application à une lumière infrarouge afin d'échauffer celle-ci, et un moyen d'émission d'un rayonnement radioélectrique permettant d'élever la température de la surface extérieure, un ventilateur pour souffler de l'air chaud sur la surface extérieure ou une source d'ultrasons pour réchauffer la surface extérieure.

Le dispositif de chauffage peut encore comporter au moins deux composants capables de produire, lorsque mélangés, une réaction exothermique.

Le morceau de produit peut être en forme de bâtonnet et la surface extérieure être définie par l'extrémité du bâtonnet.

Le dispositif de chauffage peut comporter une source d'énergie électrique comportant une ou plusieurs piles ou accumulateurs.

Le dispositif de chauffage peut comporter un générateur électrique actionné par l'utilisateur.

Le dispositif de chauffage peut comporter des moyens permettant d'assurer un chauffage du morceau de composition à une température prédéfinie, malgré l'usure dudit morceau. Ce moyen peut comporter un organe élastiquement déformable, qui assure au contact ou un écartement constant entre la surface extérieure à chauffer et le dispositif de chauffage, en compensant l'usure du morceau de composition.

Ces moyens peuvent également comporter, le cas échéant, un capteur de température qui permet d'ajuster la puissance de chauffage, par exemple l'augmenter si la surface extérieure est plus éloignée de la source de chaleur.

## Coefficient de frottement dynamique

Pour caractériser le coefficient de frottement dynamique du produit, on peut utiliser un appareil comportant un chariot qui se déplace sur une longueur de 100 mm sur des roulements à billes.

Le bon déplacement du chariot est assuré grâce à un lien rigide à la traverse mobile d'une machine de traction et de compression (TAXT2 de la société Rheo) mise en position horizontale, par un aimant fixé à l'arrière du chariot.

Le produit S dont on cherche à évaluer le coefficient de frottement dynamique est coupé à une extrémité avec un fil de tungstène de diamètre 250 μm en déplaçant le fil relativement au stick à une vitesse de 100mm/min et perpendiculaire à son axe longitudinal de façon à avoir une surface de contact plane et parallèle à la surface de glissement W.

On lui applique une force normale Fn à la surface de glissement W à l'aide d'un poids. Ce poids est tel que la pression exercée sur la surface du produit S en contact avec W est de $7.9\ 10^{-3}$ MPa.

Le produit peut se présenter sous la forme d'un bâtonnet cylindrique de révolution.

Dans le cas où la section transversale du bâtonnet n'est pas circulaire, on fait glisser le stick dans la direction du petit axe de sa section, en déplaçant le grand axe parallèlement à lui-même.

Le coefficient de frottement est défini comme le rapport de la force tangentielle Ft appliquée sur le corps mis en mouvement dans la direction M sur la force normale Fn subie par ce même corps, comme illustré à la figure 15.

Dans un essai de frottement, on peut distinguer une première phase transitoire de mise en mouvement du système et une deuxième phase de régime continu.

Dans la première phase, la force tangentielle augmente pour atteindre un maximum qui correspond à la mise en mouvement du système. Ce maximum correspond à la force de frottement statique appelée Ft statique et permet de définir un coefficient de frottement statique (μs)

$$\mu s = Ft\ statique / Fn$$

où Fn est la force normale appliquée.

La force tangentielle Ft décroît ensuite pour atteindre généralement un régime plus stable. Le coefficient de frottement dynamique est défini dans cette phase du mouvement comme le rapport de la force de frottement dynamique (force tangentielle) sur la force normale appliquée (Fn) :

$$\mu d = Ft\ dynamique / Fn$$

Le coefficient de frottement est une grandeur sans dimension, fonction des deux surfaces en contact et des conditions de contact.

La surface de glissement est définie par de la peau artificielle, de référence « BIO SKIN Plate Black K275 » de la société MACREPOS, de largeur égale ou supérieure à celle de la section du stick.

Pour une mesure à 25 °C, l'ensemble de l'appareillage et la composition sont à 25 °C.

La peau artificielle est placée sur un support qui peut être chauffé à la température à laquelle on souhaite mesurer le coefficient de frottement dynamique. On applique par exemple sur la peau artificielle ainsi chauffée, par exemple à 45 °C si la mesure doit être effectuée à 45°C, le bâtonnet initialement à la température de 25 °C. La température de surface de la peau artificielle peut être contrôlée avec un thermomètre optique.

Dans certains modes de réalisation, le coefficient de frottement dynamique d'une composition selon l'invention est supérieur ou égal à 0,6, voire 0,7 ou 0,8, à 25°C. Le coefficient de frottement dynamique à 25°C des compositions selon l'invention peut être inférieur ou égal à 5.

Selon un mode de réalisation particulier, le stick peut avoir un diamètre de 12,7 mm au niveau de sa zone de contact avec la surface de glissement, mais d'autres valeurs sont possibles, allant par exemple de 7 mm à 50 mm.

## PROTOCOLE DE MESURE DE DURETE

Les compositions considérées selon l'invention sont relativement dures à température ambiante et, sous l'action de la chaleur, deviennent suffisamment molles pour être applicables.

La dureté peut être mesurée à 20°C par la méthode dite « du fil à couper le beurre », qui consiste à couper transversalement un bâton de produit, de préférence cylindrique de révolution, à l'aide d'un fil rigide de tungstène de diamètre 250 µm en déplaçant le fil relativement au stick à une vitesse de 100 mm/min. La dureté correspond à la force maximale de cisaillement exercée par le fil sur le stick à 20 °C, cette force étant mesurée au moyen d'un dynamomètre DFGHS 2 de la société Indelco-Chatillon. La mesure est reproduite trois fois puis moyennée.

La moyenne des trois valeurs lues au moyen du dynamomètre mentionné ci-dessus, notée Y, est donnée en grammes. Cette moyenne est convertie en Newton puis divisée par L qui représente la dimension la plus élevée traversée par le fil. Dans le cas d'un bâton cylindrique, L est égal au diamètre en mètres.

La dureté est convertie par l'équation ci-dessous :

$$(Yx\ 10^{-3}\ x9.8)/L$$

Pour une mesure à une température différente, on chauffe la totalité du stick à la température où la dureté doit être mesurée.

Selon cette méthode de mesure, la dureté à 20 °C d'exemples de composition selon l'invention est de façon préférée supérieure à 80 $Nm^{-1}$, notamment supérieure à 100 $Nm^{-1}$ ,de préférence supérieure à 120 $Nm^{-1}$

De façon préférée, la composition selon l'invention présente notamment une dureté à 20°C inférieure à 500 $Nm^{-1}$ notamment inférieure à 400 $Nm^{-1}$de préférence inférieure à 300 $Nm^{-1}$

Une composition de l'invention est cosmétiquement ou dermatologiquement acceptable, à savoir contient un milieu physiologiquement acceptable non toxique et susceptible d'être appliqué sur les lèvres d'êtres humains. Par cosmétiquement acceptable, on entend au sens de l'invention une composition d'aspect, d'odeur et de toucher agréables, convenant à une utilisation en cosmétique.

## POLYESTERS

La composition selon l'invention comprend au moins un polyester.

Un polyester convenant à l'invention peut être avantageusement obtenu par réaction d'un polyol, d'un acide polycarboxylique, d'un acide monocarboxylique non aromatique, et d'un acide monocarboxylique aromatique.

En particulier, un polyester convenant à l'invention peut être préférentiellement obtenu par réaction :

- d'un tétraol ayant de 4 à 10 atomes de carbone ;
- d'un acide saturé monocarboxylique, linéaire ou ramifié, ayant de 9 à 23 atomes de carbone ;
- d'un diacide carboxylique cyclique ayant de 6 à 12 atomes de carbone ; et
- d'un acide monocarboxylique aromatique avant de 7 à 11 atomes de carbone.

De façon avantageuse, un polyester de l'invention peut être obtenu par réaction :

- de 10 à 30 % en poids d'un tétraol ayant de 4 à 10 atomes de carbone ;
- de 40 à 80 % en poids d'un acide saturé monocarboxylique, linéaire ou ramifié, ayant de 9 à 23 atomes de carbone ;
- de 5 à 30 % en poids d'un diacide carboxylique cyclique ayant de 6 à 12 atomes de carbone ;
- de 0,1 à 10 % en poids d'un acide monocarboxylique aromatique ayant de 7 à 11 atomes de carbone,

les teneurs étant exprimées en pourcentage en poids par rapport au poids total du polyester.
Un polyester utilisé selon l'invention comprend un tétraol. On entend par tétraol un polyol comprenant 4 groupes hydroxyles.
Un tétraol utilisé pour la préparation du polyester est avantageusement un composé hydrocarboné, linéaire, ramifié et/ou cyclique, saturé ou insaturé, comprenant de 4 à 10 atomes de carbone, et pouvant comprendre en outre un ou plusieurs atomes d'oxygène intercalés dans la chaîne (fonction éther). On peut bien évidemment utiliser un mélange de tels tétraols.
Un tétraol peut être en particulier un composé hydrocarboné saturé, linéaire ou ramifié, comprenant 4 à 10 atomes de carbone.
Un tétraol peut être choisi parmi le pentaérythritol ou tétraméthylolméthane, l'érythritol, le diglycérol ou le ditriméthylolpropane.
De préférence, le tétraol est choisi parmi le pentaérythritol et le diglycérol.
Plus préférentiellement encore, le tétraol peut être le pentaérythritol.
La teneur en tétraol, ou en mélange de tétraol, peut représenter de 10 à 30 % en poids, notamment 12 à 25 % en poids, et mieux de 14 à 22 % en poids par rapport au poids total du polyester.
Un polyester utilisé selon l'invention comprend également un acide saturé monocarboxylique, linéaire ou ramifié, ayant de 9 à 23 atomes de carbone, et notamment 12 à 22 atomes de carbone.
Par acide monocarboxylique saturé, on entend un composé de formule RCOOH, dans laquelle R est un radical hydrocarboné saturé, linéaire ou ramifié, comprenant de 8 à 22 atomes de carbone, notamment de 11 à 21 atomes de carbone. On peut bien évidemment utiliser un mélange de tels acides monocarboxyliques.
Parmi les acides monocarboxyliques saturés susceptibles d'être employés, on peut citer, seul ou en mélange, l'acide nonanoïque, l'acide isononanoïque ou acide pélargonique, l'acide décanoïque ou acide caprique, l'acide laurique, l'acide tridécanoïque ou acide tridécylique, l'acide myristique, l'acide palmitique, l'acide stéarique, l'acide isostéarique, l'acide arachidique, et l'acide béhénique.
De préférence, on peut utiliser l'acide laurique, l'acide myristique, l'acide isononanoïque, l'acide nonanoïque, l'acide palmitique, l'acide isostéarique, l'acide stéarique, l'acide béhénique, et leurs mélanges.
Préférentiellement, on utilise l'acide isostéarique ou l'acide stéarique.
Lorsque l'acide monocarboxylique saturé est liquide à température ambiante, il conduit généralement à un polyester liquide à température ambiante.
Comme acide monocarboxylique liquide, on peut citer l'acide nonanoïque, l'acide isononanoïque, l'acide isostéarique.
Lorsque l'acide monocarboxylique saturé est solide à température ambiante, il conduit généralement à un polyester solide à température ambiante.
Comme acide monocarboxylique solide, on peut citer l'acide décanoïque, l'acide laurique, l'acide tridécanoïque, l'acide myristique, l'acide palmitique, l'acide stéarique, l'acide arachidique, l'acide béhénique.
La teneur en acide monocarboxylique saturé, ou le mélange desdits acides, représente de 40 à 80 % en poids, notamment de 42 à 75 % en poids, voire 45 à 70 % en poids, et mieux 50 à 65 % en poids du poids total du polyester.
Le polyester utilisé selon l'invention comprend également un diacide carboxylique cyclique ayant de 6 à 12 atomes de carbone, notamment ayant 8 atomes de carbone. Le diacide carboxylique cyclique peut être aromatique ou non aromatique. Le diacide carboxylique cyclique est de préférence aromatique.
On peut bien évidemment utiliser un mélange de tels diacides carboxyliques cycliques.
Un diacide carboxylique cyclique peut être choisi parmi l'acide cyclopropanedicarboxylique, l'acide cyclohexanedicarboxylique, l'acide cyclobutanedicarboxylique, l'acide phtalique, l'acide téréphtalique, l'acide isophtalique, l'acide tétrahydrophtalique, l'acide naphtalène-2,3-dicarboxylique, l'acide naphtalène-2,6-dicarboxylique, ou leurs mélanges.
De préférence, le diacide carboxylique cyclique est choisi parmi l'acide phtalique, l'acide téréphtalique, l'acide isophtalique. L'acide phtalique peut être avantageusement mis en oeuvre sous sa forme anhydride.

Préférentiellement, le diacide carboxylique cyclique est l'acide isophtalique.

Un diacide carboxylique cyclique, ou un mélange de tels diacides, peut représenter de 5 à 30 % en poids, et de préférence de 15 % à 25 % en poids du poids total du polyester.

Un polyester utilisé selon l'invention comprend également un acide monocarboxylique aromatique ayant de 7 à 11 atomes de carbone.

Par acide monocarboxylique aromatique, on entend un composé de formule R'COOH, dans laquelle R' est un radical hydrocarboné aromatique, comprenant de 6 à 10 atomes de carbone ; R' est en particulier un radical phényle, éventuellement substitué par 1 à 3 radicaux alkyle comprenant de 1 à 4 atomes de carbone.

On peut bien évidemment utiliser un mélange de tels acides monocarboxyliques aromatiques.

L'acide monocarboxylique aromatique peut être choisi parmi l'acide benzoïque et l'acide 4-tert-butyl-benzoïque.

L'acide monocarboxylique aromatique est de préférence l'acide benzoïque.

Ledit acide monocarboxylique aromatique, ou le mélange desdits acides, représente de 0,1 à 10 % en poids, notamment 0,5 à 9,95 % en poids, mieux encore de 1 à 9,5 % en poids, voire de 1,5 à 8 % en poids du poids total du polyester.

Selon un mode de réalisation préféré, ledit polyester est obtenu par réaction :

- de 12 à 25 % en poids d'un tétraol ayant de 4 à 10 atomes de carbone ;
- de 40 à 75 % en poids d'un acide saturé monocarboxylique, linéaire ou ramifié, ayant de 9 à 23 atomes de carbone ;
- de 15 à 25 % en poids d'un diacide carboxylique cyclique ayant de 6 à 12 atomes de carbone
- de 0,5 à 9,95 % en poids d'un acide monocarboxylique aromatique ayant de 7 à 11 atomes de carbone,

les teneurs étant exprimées en pourcentage en poids par rapport au poids total du polyester.

Selon un autre mode de réalisation préféré, ledit polyester est obtenu par réaction :

- de 14 à 22 % en poids d'un tétraol ayant de 4 à 10 atomes de carbone ;
- de 45 à 70 % en poids d'un acide saturé monocarboxylique, linéaire ou ramifié, ayant de 9 à 23 atomes de carbone ;
- de 15 à 25 % en poids d'un diacide carboxylique cyclique ayant de 6 à 12 atomes de carbone ;
- de 1 à 9,5 % en poids d'un acide monocarboxylique aromatique ayant de 7 à 11 atomes de carbone,

les teneurs étant exprimées en pourcentage en poids par rapport au poids total du polyester.

Selon un autre mode de réalisation préféré, ledit polyester est obtenu par réaction :

- de 14 à 22 % en poids d'un tétraol ayant de 4 à 10 atomes de carbone ;
- de 50 à 65 % en poids d'un acide saturé monocarboxylique, linéaire ou ramifié, ayant de 9 à 23 atomes de carbone ;
- de 15 à 25 % en poids d'un diacide carboxylique cyclique ayant de 6 à 12 atomes de carbone ;
- de 1,5 à 8 % en poids d'acide monocarboxylique aromatique ayant de 7 à 11 atomes de carbone,

les teneurs étant exprimées en pourcentage en poids par rapport au poids total du polyester.

Dans un mode de réalisation préféré du polyester utilisé selon l'invention, l'acide monocarboxylique aromatique est présent en quantité molaire inférieure ou égale à celle de l'acide saturé monocarboxylique linéaire ou ramifié; notamment le rapport entre le nombre de mole d'acide monocarboxylique aromatique et le nombre de mole d'acide saturé monocarboxylique linéaire ou ramifié varie de 0,08 à 0,70. Ledit rapport pondéral varie de préférence de 0,10 à 0,60, et encore préférentiellement de 0,12 à 0,40.

Selon un mode de réalisation de l'invention, un polyester de l'invention peut être choisi parmi les polyesters d'acide benzoïque/acide isophtalique/acide isostéarique/pentaérythritol, les polyesters d'acide benzoïque/acide isophtalique/acide stéarique/pentaérythritol, et leurs mélanges

Ces monomères sont mis en oeuvre notamment selon les plages de concentrations en monomères décrites précédemment.

De préférence, le polyester présente :

- un indice d'acide, exprimé en mg d'hydroxyde de potassium par gramme de polyester, supérieur ou égal à 1; notamment compris entre 2 et 30, et encore mieux compris entre 2,5 et 15; et/ou
- un indice d'hydroxyle, exprimé en mg d'hydroxyde de potassium par gramme de polyester, supérieur ou égal à 40; notamment compris entre 40 et 120, et encore mieux compris entre 40 et 80.

Ces indices d'acide et d'hydroxyle peuvent être aisément déterminés par l'homme du métier par les méthodes analytiques habituelles.

De préférence, un polyester de l'invention présente une masse moléculaire moyenne en poids (Mw) comprise

entre 3000 et 1 000 000 g/mol, voire entre 3000 et 300 000 g/mol.

Le poids moléculaire moyen peut être déterminé par chromatographie par perméation sur gel ou par diffusion de la lumière, selon la solubilité du polymère considéré.

De préférence, un polyester de l'invention présente une viscosité, mesurée à 110 °C, comprise entre 20 et 4000 mPa.s, notamment entre 30 et 3500 mPa.s, voire entre 40 et 3000 mPa.s et encore mieux entre 50 et 2500 mPa.s. Cette viscosité est mesurée de la manière décrite ci-après.

Selon un mode de réalisation préférée, le polyester peut être sous forme liquide à température ambiante. Un polyester liquide peut avoir une masse moléculaire moyenne en poids (Mw) allant de 40 000 à 1 000 000 g/mol, de préférence allant de 50 000 à 300 000 g/mol.

Un polyester liquide peut avoir une viscosité, mesurée à 110 °C , allant de 1000 à 4000 mPa.s, et de préférence allant de 1500 à 3000 mPa.s.

En particulier, un polyester liquide peut être un polyester d'acide benzoïque/acide isophtalique/acide isostéarique/pentaérythritol, ces monomères étant notamment présents selon les plages de concentrations en monomères décrites précédemment.

Selon un autre mode de réalisation, le polyester peut également être sous forme solide à température ambiante. Un polyester solide peut avoir une masse moléculaire moyenne en poids (Mw) allant de 3 000 à 30 000 g/mol, de préférence allant de 8 000 à 15 000 g/mol.

Le polyester solide peut avoir une viscosité, mesurée à 80 °C, allant de 20 à 1 000 mPa.s, et de préférence allant de 50 à 600 mPa.s.

En particulier, un polyester solide peut être un polyester d'acide benzoïque/acide isophtalique/acide stéarique/ pentaérythritol, ces monomères étant notamment présents selon les plages de concentrations en monomères décrites précédemment.

Un polyester de l'invention peut être préparé selon le procédé de synthèse décrit dans la demande EP-A-1 870 082.

La viscosité d'un polyester de l'invention peut être mesurée de la manière décrite ci-après :

La viscosité à 80 °C ou à 110 °C d'un polyester est mesurée à l'aide d'un viscosimètre à cône plan de type BROOKFIELD CAP 1000+.

Le cône-plan adapté est déterminé par l'homme du métier, sur la base de ses connaissances; notamment :

- entre 50 et 500 mPa.s, on peut utiliser un cône 02,
- entre 500 et 1000 mPa.s : cône 03,
- entre 1000 et 4000 mPa.s : cône 05, et
- entre 4000 et 10000 mPa.s : cône 06.

La quantité de polyester, présent dans une composition de l'invention peut varier de 1 à 60 % en poids, de préférence de 2 à 50 % en poids, notamment de 3 à 45 % en poids, voire de 4 à 35 % en poids, et mieux de 5 à 30 % en poids par rapport au poids total de la composition.

Un polyester convenant à l'invention peut être aisément véhiculable dans les milieux solvants ou huileux cosmétiques, notamment les huiles, les alcools gras et/ou les esters gras.

Un polyester de l'invention peut être préparé aisément, en une seule étape de synthèse, et sans produire de déchets, et ceci à faible coût.

Un polyester convenant à l'invention peut être avantageusement branché (ramifié) afin de générer un réseau par enchevêtrement de chaînes polymériques, et donc d'obtenir les propriétés recherchées, notamment en terme de tenue améliorée, de brillance améliorée, et en terme de solubilité.

Selon un mode de réalisation, une composition de l'invention peut comprendre au moins deux polyesters distincts l'un de l'autre.

De façon préféré, le polyester, est présent dans une composition selon l'invention en une teneur variant de 1 à 60 % en poids, de préférence de 5 à 50 % en poids, notamment de 10 à 45 % en poids, voire de 10 à 35 % en poids, et mieux de 15 à 30 % en poids par rapport au poids total de la composition.

## PHASE GRASSE

Corps Gras solide :

La composition selon l'invention comprend au moins un corps gras solide choisi parmi les cires et les corps gras pâteux, et leur mélange.

**Cire**

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

Selon un mode de réalisation de l'invention, les cires d'épilation sont exclues, à titre de cires convenant à la mise en oeuvre de l'invention.

En particulier, les cires convenant à l'invention peuvent présenter un point de fusion supérieur ou égal à 45 °C, et en particulier supérieur ou égal à 55°C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines (telle que la celle commercialisée sous la référence Microwax HW par la société Paramelt), les paraffines et l'ozokérite; les cires de polyéthylène telles que celles commercialisées sous la dénomination Performalene 500-L et Performalene 400 par la société New Phase Technologies, les cires obtenues par la synthèse de Fisher-Tropsch.

On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone ($C_{30-45}$ ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.

Comme cire, on peut utiliser un (hydroxystéaryloxy)stéarate d'alkyle en $C_{20}$-$C_{40}$ (le groupe alkyle comprenant de 20 à 40 atomes de carbone), seul ou en mélange. Une telle cire est notamment vendue sous les dénominations « Kester Wax K 82 P® », « Hydroxypolyester K 82 P® » et « Kester Wax K 80 P® », et « Kester Wax K 82 H® » par la société KOSTER KEUNEN.

Comme microcires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 1145® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 2505® par la société MICRO POWDERS et les micro cires de polytétrafluoroéthylène telles que celles commercialisées sous les dénominations de Microslip 519® et 519 L® par la société MICRO POWDERS.

La composition selon l'invention peut comprendre une teneur en cires allant de 0,1 à 50 % en poids, en particulier de 0,1 à 45 % en poids, par exemple 2 à 35 % en poids, 4 à 30 % en poids, ou encore selon certains modes de réalisation de 4 à 15 % en poids, par rapport au poids total de la composition.

**Composés pâteux**

La composition selon l'invention peut également comprendre, au moins un composé pâteux.

Par "pâteux" au sens de la présente invention, on entend un composé gras lipophile à changement d'état solide/liquide réversible, présentant à l'état solide une organisation cristalline anisotrope, et comportant à la température de 23 °C une fraction liquide et une fraction solide.

En d'autres termes, la température de fusion commençante du composé pâteux peut être inférieure à 23 °C. La fraction liquide du composé pâteux mesurée à 23 °C peut représenter 9 à 97 % en poids du composé. Cette fraction liquide à 23 °C représente de préférence entre 15 et 85 %, de préférence encore entre 40 et 85 % en poids.

La fraction liquide en poids du composé pâteux à 23 °C est égale au rapport de l'enthalpie de fusion consommée à 23 °C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10 °C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23 °C est la quantité d'énergie absorbée par l'échantillon pour passer de l'état solide à l'état qu'il présente à 23 °C constitué d'une fraction liquide et d'une fraction solide.

La fraction liquide du composé pâteux mesurée à 32 °C représente de préférence de 30 à 100 % en poids du composé, de préférence de 50 à 100 %, de préférence encore de 60 à 100 % en poids du composé. Lorsque la fraction liquide du composé pâteux mesurée à 32 °C est égale à 100 %, la température de la fin de la plage de fusion du composé pâteux est inférieure ou égale à 32 °C.

La fraction liquide du composé pâteux mesurée à 32 °C est égale au rapport de l'enthalpie de fusion consommée à 32 °C sur l'enthalpie de fusion du composé pâteux. L'enthalpie de fusion consommée à 32 °C est calculée de la même façon que l'enthalpie de fusion consommée à 23 °C.

Le composé pâteux est de préférence choisi parmi les composés synthétiques et les composés d'origine végétale. Un composé pâteux peut être obtenu par synthèse à partir de produits de départ d'origine végétale.

Le composé pâteux peut avantageusement être choisi parmi :

i) la lanoline et ses dérivés,
ii) les composés siliconés polymères ou non,
iii) les composés fluorés polymères ou non,
iv) les polymères vinyliques, notamment :

- les homopolymères d'oléfines et les copolymères d'oléfines,,
- les homopolymères et copolymères de diènes hydrogénés,
- les oligomères linéaires ou ramifiés, homo ou copolymères de (méth)acrylates d'alkyles ayant de préférence un groupement alkyle en $C_8$-$C_{30}$,
- les copolymères de vinylpyrrolidone/eicosène (nom INCI VP/eicosene copolymer), par exemple vendu par la société ISP sous le nom commercial Ganex V220F® ;
- les oligomères homo et copolymères de vinyléthers ayant des groupements alkyles en $C_8$-$C_{30}$,

v) les polyéthers liposolubles résultant de la polyéthérification entre un ou plusieurs diols en $C_2$-$C_{100}$, de préférence en $C_2$-$C_{50}$,
vi) les esters,
vii) et leurs mélanges.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment du polyacyladipate-2 de bis-diglycéryle, notamment tel que commercialisé sous la marque Softisan 649® par la société Sasol,
- le propionate d'arachidyle commercialisé sous la marque Waxenol 801 par Alzo,

- les esters de phytostérol,
- les triglycérides d'acides gras et leurs dérivés,
- les esters de pentaérythritol,
- les polyesters non réticulés résultant de la polycondensation entre un acide dicarboxylique ou un polyacide carboxylique linéaire ou ramifié en $C_4$-$C_{50}$ et un diol ou un polyol en $C_2$-$C_{50}$,
- les esters aliphatiques d'ester résultant de l'estérification d'un ester d'acide hydroxycarboxylique aliphatique par un acide carboxylique aliphatique,
- les esters résultant de l'estérification d'un acide aliphatique et d'un ester aliphatique hydroxylé. Ces esters peuvent résulter de l'estérification a) d'un acide aliphatique monocarboxylique ou polycarboxylique, et b) d'un ester aliphatique hydroxylé, notamment un ester d'acide hydroxy carboxylique.
- les esters de dimère diol et dimère diacide, le cas échéant, estérifiés sur leur(s) fonction(s) alcool(s) ou acide(s) libre(s) par des radicaux acides ou alcools tels que le bis-béhényl/isostéaryl/phytostéryl dimère dilinoléyl notamment commercialisé sous le nom commercial Plandool-G® par la société Nippon Fine Chemical ,
- et leurs mélanges.

Parmi les composés pâteux, on choisira de préférence le bis-béhényl/isostéaryl/phytostéryl dimère dilinoléyl, le bis-diglycéryl polyacyladipate-2, l'huile de ricin hydrogénée dimère dilinoléate par exemple le RISOCAST-DA-L vendu par KOKYU ALCOHOL KOGYO, l'isostéarate d'huile de ricin hydrogénée par exemple le SALACOS HCIS (V-L) vendu par NISSHIN OIL ou leur mélange.

La teneur en composé pâteux peut aller de 5 à 90 % en poids, notamment de 5 à 50 % en poids, voire dans certains modes de réalisation de 5 à 35 % en poids, par rapport au poids total de la composition.

<u>**HUILE**</u>

Selon un mode de réalisation préféré, la composition selon l'invention comprend au moins une huile.

Par « huile », on entend un composé non aqueux, liquide à température ambiante (25 °C) et pression atmosphérique (760 mm de Hg).

L'huile peut être volatile ou non volatile.

Au sens de l'invention, une huile volatile présente, à température ambiante (25 °C) et pression atmosphérique (760 mm de Hg) une pression de vapeur allant de 0,02 mm à 300 mm de Hg (2,66 Pa à 40 000 Pa) et mieux allant de 0,1 à 90 mm de Hg (13 Pa à 12 000 Pa). Les huiles non volatiles correspondent alors à une pression de vapeur inférieure à 0,02 mm de Hg (2,66 Pa), et mieux, inférieure à $10^{-3}$ mm de Hg (0, 13 Pa).

L'huile volatile peut être une huile siliconée, hydrocarbonée ou fluorée.

Selon une première variante l'huile volatile peut-être une huile siliconée volatile.

Par « huile siliconée », on entend une huile comprenant au moins un atome de silicium, et notamment comprenant des groupes Si-O.

L'huile volatile siliconée utilisable dans l'invention peut être choisie parmi les huiles siliconées ayant un point éclair allant de 40 °C à 150 °C, de préférence ayant un point éclair supérieur à 55°C et inférieur ou égal à 105 °C, et préférentiellement allant de 65 °C à 95 °C. Le point éclair est en particulier mesuré selon la norme iso 3679.

L'huile siliconée volatile peut être choisie parmi les huiles siliconées linéaires ou cycliques telles que les polydiméthylsiloxanes (PDMS) linéaires ou cycliques ayant de 3 à 7 atomes de silicium.

A titre d'exemple de telles huiles, on peut citer l'octyltriméthicone, l'hexyltriméthicone, la décaméthylcyclopentasiloxane (cyclopentasiloxane ou D5), l'octaméthylcyclotétrasiloxane (cyclotétradiméthylsiloxane ou D4), la dodécaméthylcyclohexasiloxane (D6), la décaméthyltétrasiloxane (L4), KF 96 A de Shin Etsu, les polydiméthysiloxanes telles que celles commercialisées sous la référence DC 200 (1,5 cSt), DC 200 (5 cSt), DC 200 (3 cSt) par Dow Corning.

Selon une autre variante l'huile volatile peut-être une huile hydrocarbonée volatile.

Par « huile hydrocarbonée », on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Les huiles (également appelées solvants) hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme autres solvants (huiles) hydrocarbonés volatiles utilisables dans la composition selon l'invention, on

peut également citer, les cétones liquides à température ambiante tels que méthyléthylcétone, l'acétone; les esters à chaîne courte (ayant de 3 à 8 atomes de carbone au total) tels que l'acétate d'éthyle, l'acétate de méthyle, l'acétate de propyle, l'acétate de n-butyle ; les éthers liquides à température ambiante tels que le diéthyléther, le diméthyléther ou le dichlorodiéthyléther; les alcools et notamment les monoalcools inférieurs linéaires ou ramifiés ayant de 2 à 5 atomes de carbone comme l'éthanol, l'isopropanol ou le n-propanol.

Selon un mode de réalisation préféré, la composition mise en oeuvre selon l'invention comprend une teneur en huile volatile inférieure à 5% en poids, de préférence allant de 0 % à 30% en poids par rapport au poids total de la composition, ou encore est exempte huile volatile.

Selon un mode de réalisation préféré, la composition mise en oeuvre selon l'invention est exempte d'huile volatile.

## Huile non volatile

Selon un mode de réalisation préféré, la composition mise en oeuvre selon l'invention comprend au moins une huile non volatile.

Par "huile non volatile", on entend une huile restant sur les matières kératiniques à température ambiante et pression atmosphérique au moins plusieurs heures et ayant notamment une pression de vapeur inférieure à $10^{-3}$ mm de Hg (0,13 Pa). On peut également définir une huile non volatile comme ayant une vitesse d'évaporation telle que dans les conditions définies précédemment, la quantité évaporée au bout de 30 minutes est inférieure à 0,07mg/$cm^2$.

L'huile non volatile peut être une huile hydrocarbonée, une huile siliconée ou fluorée.

De façon préférée, la composition selon l'invention comprend une teneur en huile non volatile allant de 1 à 90 % en poids, de préférence de 5 à 80 % en poids, mieux de 10 à 75 % en poids, mieux encore de 20 à 70 % en poids, et encore mieux de 30 à 70% en poids, par rapport au poids total de la composition.

Selon un premier mode de réalisation, la composition mise en oeuvre dans l'invention comprend au moins une huile non volatile ayant une masse molaire inférieure à 500 g/mole.

Ces huiles peuvent être d'origine végétale, minérale ou synthétique.

A titre d'exemple d'huile non volatile utilisable dans l'invention, on peut citer :

- les huiles hydrocarbonées végétales telles que les triglycérides liquides d'acides gras ayant de 4 à 10 atomes de carbone comme les triglycérides des acides heptanoïque ou octanoïque ; l'huile de jojoba ou le squalane ;
- les alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles ester telles que
  - les esters d'acide gras, en particulier de 4 à 22 atomes de carbone, et notamment d'acide octanoïque, d'acide heptanoïque, d'acide lanolique, d'acide oléique, d'acide laurique, d'acide stéarique comme le dioctanoate de propylène glycol, le monoisostéarate de propylène glycol, le diheptanoate de néopentylglycol,
  - les esters de synthèse comme les huiles de formule $R_1COOR_2$ dans laquelle $R_1$ représente le reste d'un acide gras linéaire ou ramifié comportant de 4 à 25 atomes de carbone et $R_2$ représente une chaîne hydrocarbonée notamment ramifiée contenant de 4 à 25 atomes de carbone à condition que $R_1$ + $R_2$ (c'est-à-dire la somme des atomes de carbone de R1 et de R2) soit compris entre 16 et 30, comme par exemple l'huile de Purcellin (octanoate de cétostéaryle), l'isononanoate d'isononyle, le benzoate d'alcool en $C_{12}$ à $C_{15}$, le palmitate d'éthyl 2-hexyle, le néopentanoate d'octyledodécyle, le stéarate d'octyl-2 dodécyle, l'érucate d'octyl-2 dodécyle, l'isostéarate d'isostéaryle, le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, le palmitate de 2-éthyl-hexyle, le laurate de 2-hexyl-décyle, le palmitate de 2-octyl-décyle, le myristate de 2-octyldodécyle, le succinate de 2-diéthyl-hexyle;
  - les esters hydroxylés comme le lactate d'isostéaryle, l'octylhydroxystéarate, l'hydroxystéarate d'octyldodécyle, le stéarate de glycérine; le diisononanoate de diéthylèneglycol ; et
- les huiles siliconées de poids moléculaire inférieur à 500g/mole, comme les polydiméthylsiloxanes (PDMS), linéaires ou cycliques; les polydiméthylsiloxanes comportant des groupements alkyle, alcoxy ou phényle, pendant ou en bout de chaîne siliconée, groupements ayant de 2 à 24 atomes de carbone ;
- les acides gras ayant de 12 à 26 atomes de carbone comme l'acide oléïque ;
- et leurs mélanges.

Selon un premier mode de réalisation, la composition mise en oeuvre selon l'invention est exempte d'huile de poids moléculaire inférieur à 500 g/mol.

Selon un autre mode de réalisation, la composition mise en oeuvre selon l'invention comprend une teneur en huile non volatile de poids moléculaire inférieur à 500 g/mole allant de 0,1 à 80 % en poids, de préférence de 0,1 à 60 % en poids, mieux de 0,1 à 50 % en poids et encore mieux de 1 à 40 % en poids, par rapport au poids total de la composition.

## HUILE BRILLANTE

De façon préférée, la composition selon l'invention comprend au moins une huile non volatile de poids moléculaire supérieur à 500 g/mole, appelée « huile brillante ».

L'huile brillante a de préférence une masse molaire élevée, à savoir allant de 500 à 100 000 g/mol, de préférence allant de 500 à 25 000 g/mol, ou encore de 500 à 10 000 g/mol.

De préférence, l'huile brillante a un indice de réfraction supérieur ou égal à 1,45 et notamment allant de 1,45 à 1,65.

L'huile brillante utilisable dans la présente invention peut être choisie parmi :

- les polymères lipophiles tels que :
- les polybutylènes tels que L'INDOPOL H-100 (de masse molaire ou MW=965 g/mol), L'INDOPOL H-300 (MW=1340 g/mol), L'INDOPOL H-1500 (MW=2160g/mol) commercialisés ou fabriqués par la société AMOCO,
- les polyisobutylènes hydrogénés tels que le PANALANE H-300 E commercialisés ou fabriqué par la société AMOCO (MW =1340 g/mol), le VISEAL 20000 commercialisé ou fabriqué par la société SYNTEAL (MW=6000 g/mol), le REWOPAL PIB 1000 commercialisé ou fabriqué par la société WITCO (MW=1000 g/mol),
- les polydécènes et les polydécènes hydrogénés tels que : le PURESYN 10 (MW=723 g/mol), le PURESYN 150 (MW=9200 g/mol) commercialisés ou fabriqués par la société MOBIL CHEMICALS,
- les copolymères de la vinylpyrrolidone tels que : le copolymère vinylpyrrolidone/1-héxadécène, ANTARON V—216 commercialisé ou fabriqué par la société ISP (MW=7300 g/mol),
- les esters tels que :
    - les esters du pentaérythritol ;
    - les esters d'acides aromatiques et d'alcools comprenant 4 à 22 atomes de carbone, notamment le trimellitate de tridécyle.
    - les esters hydroxylés tels que le diisostéarylmalate, ou le triisostéarate de polyglycérol-2 (MW=965 g/mol),
    - les esters aromatiques tels que le tridécyl trimellitate (MW=757 g/mol),
    - les esters d'alcool gras ou d'acides gras ramifiés en $C_{24}$-$C_{28}$ tels que ceux décrits dans la demande EP-A-0 955 039, et notamment le citrate de triisoarachidyle (MW=1033,76 g/mol), le tétraisononanoate de pentaérythrityle (MW=697 g/mol), le triisostéarate de glycéryle (MM=891 g/mol), le tri décyl-2 tétradécanoate de glycéryle (MW=1143 g/mol), le tétraisostéarate de pentaérythrityle (MW=1202 g/mol), le tétraisostéarate de polyglycéryle —2 (MW=1232 g/mol) ou encore le tétra décyl -2 tétradécanoate de pentaérythrityle (MW=1538 g/mol),
    - les esters de dimère diol et de dimère diacide de formule générale HO-$R^1$-(-OCO-$R^2$-COO-$R^1$-)$_h$-OH, dans laquelle :

        $R^1$ représente un reste de dimère diol obtenu par hydrogénation du diacide dilinoléique $R^2$ représente un reste de diacide dilinoléique hydrogéné, et
        h représente un entier variant de 1 à 9,
        notamment les esters de diacides dilinoléiques et de dimères diols dilinoléiques commercialisés par la société NIPPON FINE CHEMICAL sous la dénomination commerciale LUSPLAN DD-DA5® et DD-DA7®,

        - les huiles siliconées telles que les silicones phénylées,
        - et leurs mélanges.

De façon préférée lorsque l'huile brillante est hydrocarbonée, elle a de préférence une masse molaire allant de 500 à 10 000 g/mol, de préférence de 500 à 7500 g/mol.

De préférence, l'huile brillante a un indice de réfraction supérieur ou égal à 1,45 et notamment allant de 1,45 à 1,65.

De façon préférée, l'huile de poids moléculaire supérieur à 500 g/mol peut représenter de 1 à 90 % en poids, de préférence de 5 à 80 % en poids, mieux de 10 à 75 % en poids, mieux encore de 20 à 70 % en poids, et encore mieux de 30 à 70% en poids par rapport au poids total de la composition.

Selon un mode de réalisation préféré, la composition selon l'invention comprend au moins une huile siliconée phénylée (également appelée « silicone phénylée ») non volatile.

En particulier, la composition selon l'invention comprend de préférence au moins une huile siliconée phénylée

de poids moléculaire supérieur à 500 g/mole (appelée « huile brillante »). De préférence, le poids moléculaire en poids de l'huile siliconée phénylée est compris entre 500 et 10 000 g/mol, de préférence entre 500 et 7500 g/mol.

On entend par huile siliconée phénylée, un organopolysiloxane substitué par au moins un groupe phényle.

L'huile siliconée peut être choisie parmi les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

L'huile siliconée peut répondre à la formule:

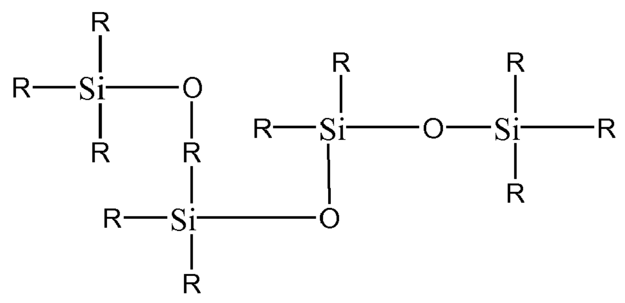

dans laquelle les groupements R représentent indépendamment les uns des autres un méthyle ou un phényle. De préférence dans cette formule, l'huile siliconée comprend au moins trois groupes phényle, par exemple au moins quatre, au moins cinq ou au moins six.

Selon un mode de réalisation préféré de l'invention, l'huile siliconée répond à la formule A1

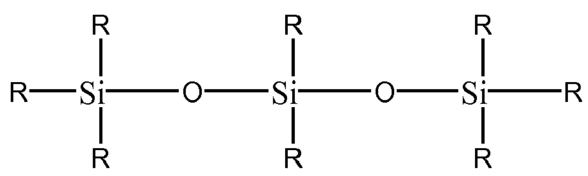

dans laquelle les groupements R représentent indépendamment les uns des autres un méthyle ou un phényle. De préférence dans cette formule, ledit organopolysiloxane comprend au moins trois groupes phenyles, par exemple au moins quatre ou au moins cinq.

Des mélanges des organopolysiloxanes phénylés décrits précédemment peuvent être utilisés.

On peut citer par exemples des mélanges d'organopolysiloxane tétraphénylé, pentaphénylé.

Selon ce mode de réalisation, l'huile siliconée répond de préférence à la formule

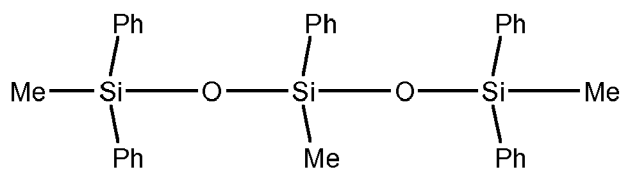

dans laquelle Me représente un groupe méthyle, et Ph représente un groupe phényle. Une telle silicone phénylée est notamment fabriquée par Dow Corning sous la référence Dow Corning 555 Cosmetic Fluid (nom INCI : trimethyl pentaphenyl trisiloxane). La référence Dow Corning 554 Cosmetic Fluid peut aussi être utilisée.

Selon un autre mode de mise en oeuvre, l'huile siliconée répond à la formule

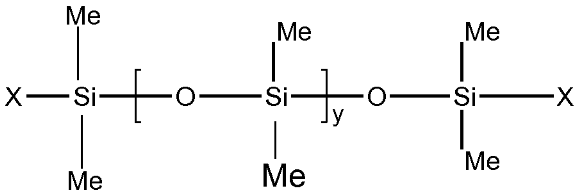

dans laquelle Me représente un groupe méthyle, y est compris entre 1 et 1 000, et X représente —CH2-CH(CH3)(Ph).

Selon un autre mode de mise en oeuvre, l'huile siliconée répond à la formule

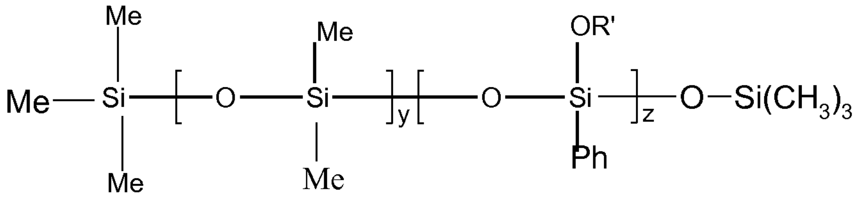

dans laquelle -OR' représente un groupe —O-SiMe3, y est compris entre 1 et 1 000 et z est compris entre 1 et 1 000.

L'huile siliconée phénylée peut être choisie parmi les silicones phénylées de formule (VI) suivante :

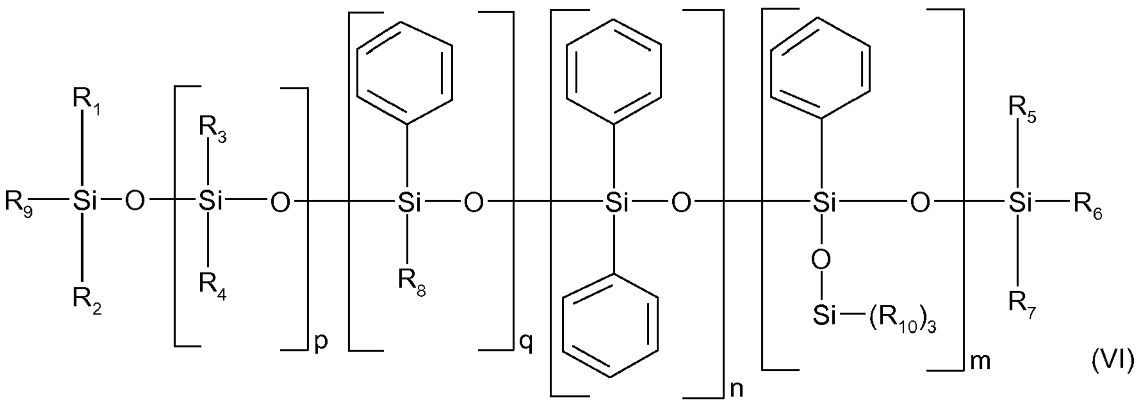

(VI)

dans laquelle

- R1 à R10, indépendamment les uns des autres, sont des radicaux hydrocarbonés, saturés ou insaturés, linéaires, cycliques ou ramifiés, en C1-C30,
- m, n, p et q sont, indépendamment les uns des autres, des nombres entiers compris entre 0 et 900, sous réserve que la somme 'm+n+q' est différente de 0.

De préférence, la somme 'm+n+q' est comprise entre 1 et 100. De préférence, la somme 'm+n+p+q' est comprise entre 1 et 900, encore mieux entre 1 et 800. De préférence, q est égal à 0.

L'huile siliconée phénylée peut être choisie parmi les silicones phénylées de formule (VII) suivante :

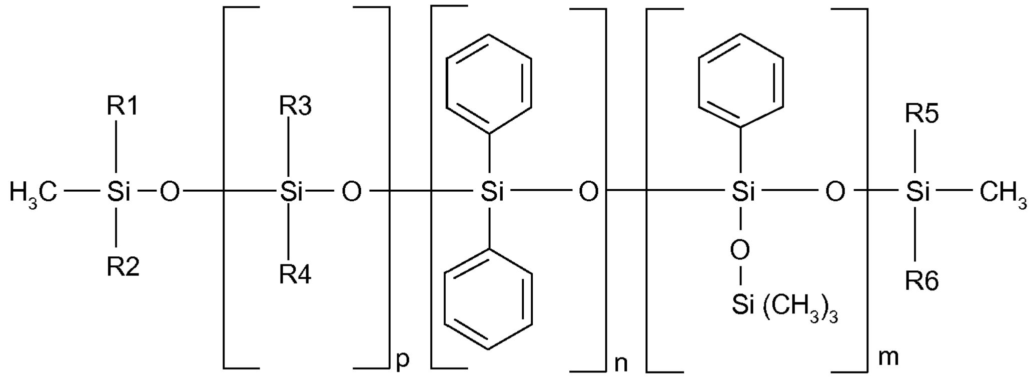

(VII)

dans laquelle :

- R1 à R6, indépendamment les uns des autres, sont des radicaux hydrocarbonés, saturés ou insaturés, linéaires cycliques ou ramifiés, en C1-C30,
- m, n et p sont, indépendamment les uns des autres, des nombres entiers compris entre 0 et 100, sous réserve que la somme 'n + m' est comprise entre 1 et 100.

De préférence, R1 à R6, indépendamment les uns des autres, représentent un radical hydrocarboné saturé, linéaire ou ramifié, en C1-C30, notamment en C1-C12, et en particulier un radical méthyle, éthyle, propyle ou butyle.
Notamment, R1 à R6 peuvent être identiques, et en outre peuvent être un radical méthyle.
De préférence, on peut avoir m=1 ou 2 ou 3, et/ou n=0 et/ou p=0 ou 1, dans la formule (VII).
On peut utiliser une huile siliconée phénylée de formule (VI) ayant une viscosité à 25°C comprise entre 5 et 1500 $mm^2/s$ (soit 5 à 1500 cSt), de préférence ayant une viscosité comprise entre 5 et 1000 $mm^2/s$ (soit 5 à 1000 cSt).
Comme huile siliconée phénylée de formule (VII), on peut utiliser notamment les phényltriméthicones telles que les huiles Belsil, notamment Belsil PDM1000 (1000cSt) et Belsil PDM 200 (200 cSt) de Wacker, l'huile Silbione 70663V30 de Rhône Poulenc (28 cSt), ou les diphényldiméthicones. Les valeurs entre parenthèses représentent les viscosités à 25°C.
L'huile siliconée non volatile peut être choisie parmi les silicones de formule :

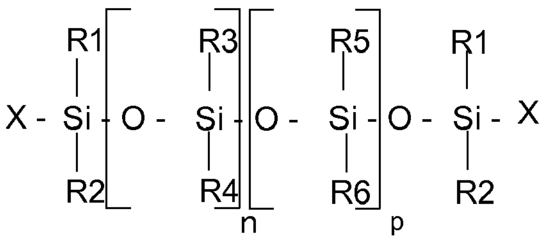

dans laquelle :

$R_1$, $R_2$, $R_5$ et $R_6$ sont, ensemble ou séparément, un radical alkyle ayant 1 à 6 atomes de carbone,
$R_3$ et $R_4$ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à l'huile une masse moléculaire en poids inférieure à 200 000 g/mol, de préférence inférieure à 150 000 g/mol et de préférence encore inférieure à 100 000 g/mol.

En particulier, on utilise de préférence à titre d'huile siliconée phénylée les phényl triméthicones, les phényl diméthicones, les phényl triméthylsiloxy diphényl siloxanes, les diphényl diméthicones, le diphényl méthyldiphényl trisiloxane, le triméthyl pentaphényl trisiloxane (notamment le 1,3,5-triméthyl 1,1,3,5,5-pentaphenyl trisiloxane commercialisé sous le nom PH-1555 HRI par Dow Corning) et leurs mélanges.
De façon préférée, l'huile siliconée phénylée de poids moléculaire supérieur à 500 g/mol (telle qu'une huile de formule A1) est présente en une teneur allant de 1 à 90 % en poids, de préférence de 5 à 80 % en poids, mieux de 10

à 75 % en poids, mieux encore de 20 à 70 % en poids, et encore mieux de 30 à 70% en poids par rapport au poids total de la composition.

De façon préférée, lorsque la composition comprend une huile siliconée phénylée, celle-ci est présente dans la composition dans un ratio pondéral huile siliconée phénylée / polyester compris entre 1 et 20, de préférence entre 1,5 et 10.

De façon préférée, la composition comprend une huile siliconée phénylée de poids moléculaire supérieur à 500 g/mol et au moins une l'huile hydrocarbonée de poids moléculaire supérieur à 500 g/mol.

Outre les composés précités, une composition selon l'invention peut également comprendre d'autres composés notamment tels que définis ci-après. Il est entendu que la quantité en ces composés annexes peut être ajustée par l'homme du métier de manière à ne pas porter préjudice à l'effet recherché dans le cadre de la présente invention.

En particulier, selon un mode de réalisation préféré, la composition mise en oeuvre selon l'invention comprend au moins un ingrédient additionnel choisi parmi les matières colorantes, les charges, les huile non volatiles, les agents structurants différents des cires ou des corps gras pâteux, les polymères semi-cristallins, polymères filmogènes et les polyamides siliconés.

**<u>Autres agents structurants</u>**

La composition selon la présente invention peut en outre comprendre d'autres agents structurants additionnel différents d'une cire telle que précédemment définie. On entend par agent structurant un composé apte à augmenter la viscosité de la composition l'incorporant. L'agent structurant additionnel permet notamment d'obtenir une composition pouvant présenter une texture allant des textures fluides à solides.

A ce titre on peut notamment citer :

- les argiles organophiles, comme les hectorites modifiées par un chlorure d'ammonium en $C_{10}$ à $C_{22}$, comme l'hectorite modifiée par du chlorure de di-stéaryl di-méthyl ammonium telle que, par exemple, celle commercialisée sous la dénomination de Bentone 38V® par la société ELEMENTIS.
- les silices pyrogénées, comme les silices pyrogénées éventuellement traitées hydrophobe en surface dont la taille des particules est inférieure à 1 μm Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

    1. des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812® par la société DEGUSSA, CAB-O-SIL TS-530® par la société CABOT,
    2. des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972®, et Aerosil R974® par la société DEGUSSA, CAB-O-SIL TS-610® et CAB-O-SIL TS-720® par la société CABOT.

- les gommes de guar alkylées (avec groupe alkyle en C1-C6), telles que celles décrites dans EP-A-708114, ou par exemple les dérivés de cellulose tels que l'éthylcellulose comme celle vendue sous la dénomination Ethocel® par la société DOW CHEMICAL ;
- Les copolymères séquencés hydrocarbonés appelés également copolymère bloc, de préférence un copolymère séquencé soluble ou dispersible dans une phase grasse liquide.

Le copolymère bloc hydrocarboné peut être notamment un copolymère dibloc, tribloc, multibloc, radial, étoile, ou leurs mélanges.

De tels copolymères blocs hydrocarbonés sont décrits dans la demande US-A-2002/005562 et dans le brevet US-A-5,221,534.

Comme copolymère dibloc, de préférence hydrogéné, on peut citer les copolymères de styrène-éthylène/propylène, les copolymères de styrène-éthylène/butadiène, les copolymères de styrène-éthylène/butylène. Des polymères diblocs sont notamment vendus sous la dénomination Kraton® G1701E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-éthylène/butylène-styrène les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

- et leurs mélanges.

De tels autres agents structurants peuvent être compris dans la composition conforme à l'invention dans une teneur comprise entre 0,5 % et 20 % en poids, notamment entre 0,5 % et 10 % en poids, par rapport au poids total de la composition.

**Polymère semi-cristallin**

La composition selon l'invention peut également comprendre avantageusement au moins un polymère semi-cristallin à structure organique dont la température de fusion est supérieure ou égale à 30 °C.
De préférence, la quantité totale de polymère(s) semi-cristallin(s) représente de 0,1 à 45 % du poids total de la composition, et mieux de 0,5 à 40 %, par exemple de 1 à 35 % en poids, et encore mieux de 1 à 20 %, ou encore de 3 à 30 %, 5 à 30 %, voire 15 à 30 %. De préférence, il représente de 2 % à 10 % en poids de la composition.
Par « polymères », on entend au sens de l'invention des composés comportant au moins 2 motifs de répétition, de préférence au moins 3 motifs de répétition et plus spécialement au moins 10 motifs répétitifs.
Par « polymère semi-cristallin », on entend au sens de l'invention, des polymères comportant une partie cristallisable et une partie amorphe et présentant une température de changement de phase réversible du premier ordre, en particulier de fusion (transition solide-liquide). La partie cristallisable est soit une chaîne latérale (ou chaîne pendante), soit une séquence dans le squelette.
Lorsque la partie cristallisable du polymère semi-cristallin est une séquence du squelette polymérique, cette séquence cristallisable est de nature chimique différente de celle des séquences amorphes; le polymère semi-cristallin est dans ce cas un copolymère séquencé par exemple du type dibloc, tribloc ou multibloc. Lorsque la partie cristallisable est une chaîne pendante au squelette, le polymère semi cristallin peut être un homopolymère ou un copolymère.
Par « composé organique » ou « à structure organique », on entend des composés contenant des atomes de carbone et des atomes d'hydrogène et éventuellement des hétéroatomes comme S, O, N, P seuls ou en association.
La température de fusion du polymère semi-cristallin est de préférence inférieure à 150 °C.
La température de fusion du polymère semi-cristallin est de préférence supérieure ou égale à 30 °C et inférieure à 100 °C. De préférence encore, la température de fusion du polymère semi-cristallin est de préférence supérieure ou égale à 30 °C et inférieure à 70 °C.
Le ou les polymères semi-cristallins selon l'invention servant sont des solides à température ambiante (25 °C) et pression atmosphérique (760 mm de Hg), dont la température de fusion est supérieure ou égale à 30 °C. Les valeurs de point de fusion correspondent au point de fusion mesuré à l'aide d'un calorimètre à balayage différentiel (D. S. C), tel que le calorimètre vendu sous la dénomination DSC 30 par la société METTLER, avec une montée en température de 5 ou 10 °C par minute. (Le point de fusion considéré est le point correspondant à la température du pic le plus endotherme du thermogramme).
Le ou les polymères semi-cristallins selon l'invention ont de préférence une température de fusion supérieure à la température du support kératinique destiné à recevoir ladite composition, en particulier la peau ou les lèvres.
Selon l'invention les polymères semi-cristallins sont avantageusement solubles dans la phase grasse, notamment à au moins 1 % en poids, à une température supérieure à leur température de fusion. En dehors des chaînes ou séquences cristallisables, les séquences des polymères sont amorphes.
Par « chaîne ou séquence cristallisable », on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère.
De préférence, le squelette polymérique des polymères semi-cristallins est soluble dans la phase grasse à une température supérieure à leur température de fusion.
De préférence, les séquences ou chaînes cristallisables des polymères semi-cristallins représentent au moins 30 % du poids total de chaque polymère et mieux au moins 40 %. Les polymères semi-cristallins à chaînes latérales cristallisables sont des homo ou des copolymères. Les polymères semi-cristallins de l'invention à séquences cristallisables sont des copolymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.
De préférence, les polymères semi-cristallins de l'invention sont d'origine synthétique.
Selon un mode de réalisation préféré, le polymère semi-cristallin est choisi parmi :

- les homopolymères et copolymères comportant des motifs résultant de la polymérisation de un ou plusieurs monomères porteurs de chaîne(s) latérale(s) hydrophobe(s) cristallisable(s),
- les polymères portant dans le squelette au moins une séquence cristallisable,
- les polycondensats de type polyester, aliphatique ou aromatique ou aliphatique/aromatique,
- les copolymères d'éthylène et de propylène préparés par catalyse métallocène.

Les polymères semi-cristallins utilisables dans l'invention peuvent être choisis en particulier parmi:

- les copolymères séquencés de polyoléfines à cristallisation contrôlée, dont les monomères sont décrits dans EP-A-0 951 897,
- les polycondensats et notamment de type polyester, aliphatique ou aromatique ou aliphatique/aromatique,
- les copolymères d'éthylène et de propylène préparés par catalyse métallocène,
- les homo- ou co-polymères portant au moins une chaîne latérale cristallisable et les homo- ou co-polymères portant dans le squelette au moins une séquence cristallisable, comme ceux décrits dans le document US-A-5,156,911,
- les homo- ou co-polymères portant au moins une chaîne latérale cristallisable en particulier à groupement(s) fluoré (s), tels que décrits dans le document WO-A-01/19333,
- et leurs mélanges.

Dans les deux derniers cas, la ou les chaînes latérales ou séquences cristallisables sont hydrophobes.

A) Polymères semi-cristallins à chaînes latérales cristallisables

On peut citer en particulier ceux définis dans les documents US-A-5,156,911 et WO-A-01/19333.
Ce sont des homopolymères ou copolymères comportant de 50 à 100 % en poids de motifs résultant de la polymérisation de un ou plusieurs monomères porteurs de chaîne latérale hydrophobe cristallisable.
Ces homo- ou co-polymères sont de toute nature du moment qu'ils présentent les conditions indiquées ci-après avec en particulier la caractéristique d'être solubles ou dispersables dans la phase grasse, par chauffage au-dessus de leur température de fusion Pf. Ils peuvent résulter :

- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.

- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées.

 a) D'une façon générale les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule X :

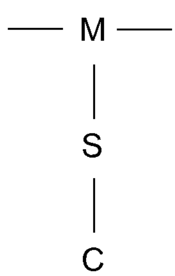

 avec M représentant un atome du squelette polymérique,
 C représentant un groupe cristallisable, et
 S représentant un espaceur

Les chaînes « -S-C » cristallisables peuvent être aliphatiques ou aromatiques, éventuellement fluorées ou

perfluorées. « C » représente notamment un groupe $(CH_2)_n$ linéaire ou ramifié ou cyclique, avec n entier allant de 12 à 40. De préférence « C » est un groupe linéaire. De préférence, « S » et « C » sont différents.

Lorsque les chaînes cristallisables sont des chaînes aliphatiques hydrocarbonées, elles comportent des chaînes alkyle hydrocarbonées à au moins 12 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyles en $C_{14}$-$C_{24}$, de préférence en $C_{16}$-$C_{22}$. Lorsqu'il s'agit de chaînes alkyle fluorées ou perfluorées, elles comportent au moins 11 atomes de carbone dont au moins 6 atomes de carbone sont fluorés.

Comme exemple d'homopolymères ou de copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en $C_{14}$-$C_{24}$, les (méth)acrylates de perfluoroalkyle avec un groupe alkyle perfluoro en $C_{11}$-$C_{15}$, les N-alkyl (méth)acrylamides avec le groupe alkyle en $C_{14}$ à $C_{24}$ avec ou sans atome de fluor, les esters vinyliques à chaînes alkyle ou perfluoro (alkyle) avec le groupe alkyle en $C_{14}$ à $C_{24}$ (avec au moins 6 atomes de fluor pour une chaîne perfluoro alkyle), les éthers vinyliques à chaînes alkyle ou perfluoro (alkyle) avec le groupe alkyle en $C_{14}$ à $C_{24}$ et au moins 6 atomes de fluor pour une chaîne perfluoro alkyle, les alpha-oléfines en $C_{14}$ à $C_{24}$ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y qui est un monomère polaire ou non polaire ou un mélange des deux :

Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un NN-dialkyl(méth)acrylamide comme par exemple le NN-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydride maléique, et leurs mélanges.

Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle en $C_1$ à $C_{10}$, comme l'$\alpha$-méthylstyrène, un macromonomère du type polyorganosiloxane à insaturation vinylique.

Par « alkyle », on entend au sens au sens de l'invention un groupement saturé notamment en $C_8$ à $C_{24}$, sauf mention exprès.

De préférence, les polymères semi-cristallins à chaîne latérale cristallisable sont des homopolymères d'alkyl (méth)acrylate ou d'alkyl(méth)acrylamide avec un groupe alkyle tel que défini ci-dessus, et notamment en $C_{14}$-$C_{24}$, des copolymères de ces monomères avec un monomère hydrophile de préférence de nature différente de l'acide (méth) acrylique comme la N-vinylpyrrolidone ou l'hydroxyéthyl (méth)acrylate et leurs mélanges.

De façon avantageuse, le ou les polymères semi-cristallins à chaîne latérale cristallisable ont une masse moléculaire moyenne en poids Mp allant de 5 000 à 1 000 000, de préférence de 10 000 à 800 000, préférentiellement de 15 000 à 500 000, de préférence encore de 100 000 à 200 000.

A titre d'exemple particulier de polymère semi-cristallin utilisable dans la composition selon l'invention, on peut citer les produits Intelimer® de la société Landec décrits dans la brochure « Intelimer® polymers », Landec IP22 (Rev. 4-97). Ces polymères sont sous forme solide à température ambiante (25 °C). Ils sont porteurs de chaînes latérales cristallisables et présentent la formule X précédente. Il s'agit de ($C_{10}$-$C_{30}$)alkyle polyacrylates, qui sont partiulièrement adaptés à titre de polymères semi-cristallins pouvant être compris dans une composition conforme à la présente invention. Ces polymères peuvent notamment présenter un poids moléculaire variant de 15 000 à 500 000, de préférence de 100 000 à 200 000.

Par exemple, on choisit le produit Intelimer® IPA 13-1 de la société Landec, qui est un polyacrylate de stéaryle de poids moléculaire d'environ 145 000 et dont la température de fusion est égale à 49 °C.

Les polymères semi-cristallins peuvent être notamment ceux décrits dans les exemples 3, 4, 5, 7, 9 du brevet US-A-5 156 911 et plus particulièrement de la copolymérisation :

- d'acide acrylique, d'hexadécylacrylate et d'isodécylacrylate dans un rapport 1/16/3,
- d'acide acrylique et de pentadécylacrylate dans un rapport 1/19,
- d'acide acrylique, d'hexadécylacrylate, éthylacrylate dans un rapport 2,5/76,5/20,
- d'acide acrylique, d'hexadécylacrylate et de méthylacrylate dans un rapport 5/85/10,
- d'acide acrylique, de polyoctadécylméthacrylate dans un rapport 2,5/97,5.

On peut aussi utiliser le polymère Structure « O » de National Starch tel que celui décrit dans le document US-A-5 736 125 de température de fusion de 44 °C

Les polymères semi-cristallins peuvent être notamment les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP tels que décrits dans le document US-A-5,519,063 ou EP-A- 550 745.

On peut aussi utiliser les polymères semi-cristallins obtenus par copolymérisation de l'acrylate de béhényle et de l'acide acrylique ou de NVP, tels que décrits dans les documents US-A-5,519,063 et EP-A- 0 550 745 et plus spécialement ceux décrits dans les exemples 3 et 4, ci-après, de préparation de polymère.

B) Les polymères portant dans le squelette au moins une séquence cristallisable

Il s'agit encore de polymères solubles ou dispersables dans la phase grasse par chauffage au-dessus de leur point de fusion Pf. Ces polymères sont notamment des copolymères séquencés constitués d'au moins deux séquences de nature chimique différente dont l'une est cristallisable.

Le polymère portant dans le squelette au moins une séquence cristallisable peut être choisi parmi les copolymères séquencés d'oléfine ou de cyclooléfine à chaîne cristallisable comme ceux issus de la polymérisation séquencée de :

- cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges, avec

- l'éthylène, le propylène, le 1-butène, le 3-méthyl-1-butène, le 1-hexène, le 4-méthyl-1-pentène, le 1-octène, le 1-décène, le 1-éicosène ou leurs mélanges,

et en particulier les copoly(éthylène/norbornène) blocs et les terpolymères (éthylène/propylène/éthylidène-norbornène), blocs. On peut aussi utiliser ceux résultants de la copolymérisation séquencée d'au moins 2 $\alpha$-oléfines en $C_2$-$C_{16}$ et mieux en $C_2$-$C_{12}$ tels que ceux cités précédemment et en particulier les bipolymères séquencés d'éthylène et d'1-octène.

Le polymère portant dans le squelette au moins une séquence cristallisable peut être choisi parmi les copolymères présentant au moins une séquence cristallisable, le reste du copolymère étant amorphe (à température ambiante). Ces copolymères peuvent, en outre, présenter deux séquences cristallisables de nature chimique différente.

Les copolymères préférés sont ceux qui possèdent à la fois à température ambiante, une séquence cristallisable et une séquence amorphe lipophile réparties séquentiellement. On peut citer par exemple les polymères possédant une des séquences cristallisables et une des séquences amorphes suivantes :

- Séquence cristallisable par nature de type polyester comme les poly(alkylène téréphtalate), ou de type polyoléfine comme les polyéthylènes ou polypropylènes.
- Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :

$\alpha$) les copolymères séquencés poly($\varepsilon$-caprolactone)-b-poly(butadiène), utilisés de préférence hydrogénés, tels que ceux décrits dans l'article D6 « Melting behavior of poly(-caprolactone)-block-polybutadiène copolymers » de S. Nojima, Macromolécules, 32, 3727-3734 (1999).

$\beta$) les copolymères séquencés poly(butylènetéréphtalate)-b-poly(isoprène) hydrogénés séquencés ou multiséquencés, cités dans l'article D7 « Study of morphological and mechanical properties of PP/PBT » de B. Boutevin et al., Polymer Bulletin, 34, 117-123 (1995).

$\gamma$) les copolymères séquencés poly(éthylène)-b-copoly(éthylène/propylène) cités dans les articles D8 « Morphology of semi-crystalline block copolymers of ethylene-(ethylene-alt-propylene) » de P. Rangarajan et al., Macromolecules, 26, 4640-4645 (1993) et D9 « Polymer agregates with crystalline cores : the system poly(ethylene)-poly(ethylene-propylene) » P. Richter et al., Macromolécules, 30, 1053-1068 (1997).

$\delta$) les copolymères séquencés poly(éthylène)-b-poly(éthyléthylène) cités dans l'article général D10 « Cristallization in block copolymers » de I.W. Hamley, Advances in Polymer Science, vol 148, 113-137 (1999).

C) Polycondensats de type polyester, aliphatique ou aromatique ou aliphatique/aromatique

Les polycondensats polyester peuvent être choisis parmi les polyesters aliphatiques. Leur masse moléculaire est de préférence supérieure ou égale à 200 et inférieure ou égal à 10000, et de préférence encore supérieure ou égale à 300 et inférieure ou égal à 5000, de préférence supérieure ou égale à 500 et supérieure ou égale à 2 000 g/mol.
Les polycondensats polyester sont en particulier choisis parmi les polycaprolactones. En particulier, les polycaprolactones peuvent être choisies parmi les homopolymères d'ε-caprolactones. L'homopolymérisation peut être initiée avec un diol, notamment un diol ayant de 2 à 10 atomes de carbone, tels que le diéthylène glycol, le 1,4-butanediol, le néopentyl glycol.
On peut utiliser par exemple les polycaprolactones, notamment celles commercialisées sous le nom de CAPA® 240 (point de fusion de 68 °C et poids moléculaire de 4000), 223 (point de fusion de 48°C et poids moléculaire de 2000), 222 (point de fusion de 48 °C et poids moléculaire de 2000), 217 (point de fusion de 44 °C et poids moléculaire de 1250), 2125 (point de fusion de 45°C et poids moléculaire de 1250), 212 (point de fusion de 45 °C et poids moléculaire de 1000), 210 (point de fusion de 38 °C et poids moléculaire de 1000), 205 (point de fusion de 39°C et poids moléculaire de 830) par la société SOLVAY, PCL-300, PCL-700 par la société UNION CARBIDE.
On peut utiliser en particulier la CAPA® 2125 dont la température de fusion est comprise entre 35 et 45 °C et dont la masse moléculaire est poids est égale à 1250.
Les polymères semi-cristallins de la composition de l'invention peuvent être ou non réticulés en partie du moment où le taux de réticulation ne gène pas leur dissolution ou dispersion dans la phase grasse par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.
De préférence, les polymères semi-cristallins de la composition selon l'invention sont non réticulés.

D) les copolymères d'éthylène et de propylène préparés par catalyse métallocène.

Le polymère semi-cristallin de la composition de l'invention peut également être un polymère obtenu par catalyse métallocène, tels que ceux décrits dans le brevet US 2007/0,031,361 dont le contenu est incorporé à titre de référence.
Ces polymères sont des copolymères d'éthylène et de propylène préparés par catalyse métallocène, c'est-à-dire par polymérisation à basse pression et en présence d'un catalyseur métallocène.
La masse moyenne en poids (Mw) de ces copolymères obtenues par catalyse métallocène décrits dans ce document est inférieure ou égale à 25 000 g/mol, elle va par exemple de 2 000 à 22 000 g/mol et mieux de 4 000 à 20 000 g/mol.
La masse moyenne en nombre (Mn) de ces copolymères obtenues par catalyse métallocène décrits dans ce document est de préférence inférieure ou égale à 15 000 g/mol, elle va par exemple de 1 000 à 12 000 g/mol, et mieux de 2 000 à 10 000 g/mol.
L'indice de polydispersité I du polymère est égal au rapport de la masse moyenne en poids Mw sur la masse moyenne en nombre Mn.
De façon préférée, l'indice de polydispersité des copolymères est compris entre 1,5 et 10, de préférence entre 1,5 et 5, de préférence entre 1,5 et 3 et mieux encore, entre 2 et 2,5.
Les copolymères peuvent être obtenus de manière connue à partir des monomères éthylène et/ou propylène par exemple par catalyse métallocène selon le procédé décrit dans le document EP 571 882 dont le contenu est incorporé à titre de référence.
Les copolymères d'éthylène et de propylène préparés par catalyse métallocène peuvent être non modifiés ou modifiés « polairement » (polar modified, c'est-à-dire modifiées de sorte qu'elles présentent des groupements polaires). Les copolymères modifiés polairement peuvent être préparés de manière connue à partir de homopolymères et les copolymères non modifiés tels que ceux décrits précédemment par oxydation avec des gaz contenant de l'oxygène, tel que l'air, ou par greffage avec des monomères polaires tels que l'acide maléique ou l'acide acrylique ou encore des dérivés de ces acides. Ces deux voies permettant de modifier polairement des polyoléfines obtenues par catalyse métallocène sont décrites respectivement dans les documents EP 890 583 et US 5,998,547 par exemple, le contenu de ces deux document étant incorporé à titre de référence.
Selon la présente invention, les copolymères d'éthylène et/ou de propylène préparés par catalyse métallocène modifiés polairement et particulièrement préférés sont les polymères modifiés de façon à ce qu'ils présentent des propriétés hydrophiles. A titre d'exemple, on peut citer des homopolymères ou des copolymères d'éthylène et/ou de propylène modifiés par la présence de groupes hydrophiles tels que l'anhydride maléique, l'acrylate, le méthacrylate,

la polyvinylpyrrolidone (PVP), etc.

Les homopolymères ou des copolymères d'éthylène et/ou de propylène modifiés par la présence de groupes hydrophiles tels que l'anhydride maléique ou l'acrylate, sont particulièrement préférés.

A titre d'exemple, on peut citer :

- les polymères de polypropylène modifiées par de l'anhydride maléique (PPMA) commercialisés par la société Clariant ou les copolymères polypropylène-éthylène-anhydride maléïque, tels que ceux commercialisés par la société Clariant sous le nom de LicoCare comme LicoCare PP207 LP3349, LicoCare CM401 LP3345, LicoCare CA301 LP 3346, et LicoCare CA302 LP 3347.

Dans le cadre d'une composition pour les lèvres, on préférera un polymère modifié polairement présentant un faible degré de cristallinité, de préférence de moins de 40%.

Les compositions peuvent également comprendre au moins un polymère fimogène.

## Polymère additionnel

Les compositions selon l'invention peuvent contenir un polymère filmogène.

Dans la présente invention, on entend par « polymère filmogène », un polymère apte à former à lui seul ou en présence d'un agent auxiliaire de filmification, un dépôt macroscopiquement continu sur les matières kératiniques. La composition peut comporter une phase aqueuse et le polymère filmogène peut être présent dans cette phase aqueuse. Dans ce cas celui-ci sera de préférence un polymère en dispersion ou un polymère amphiphile ou associatif.

Par « polymère en dispersion » on entend des polymères non solubles dans l'eau présents sous forme de particules de taille variable. Le polymère peut être réticulé ou non. La taille de particules moyenne est typiquement comprise entre 25 et 500nm, de préférence entre 50 et 200 nm. Les polymères en dispersion aqueuse suivants peuvent être utilisés : Ultrasol 2075 de Ganz Chemical, Daitosol 5000AD de Daito Kasei, Avalure UR 450 de Noveon, DYNAMX de National Starch, Syntran 5760 de Interpolymer, Acusol OP 301 de Rohm&Haas, Neocryl A 1090 de Avecia.

Les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer, Soltex OPT par la société ROHM & HAAS, les dispersions aqueuses de polymères acryliques ou styrène/acrylique vendues sous le nom de marque JONCRYL® par la société JOHNSON POLYMER ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Sancure 861®, Sancure 878® et Sancure 2060® par la société GOODRICH, Impranil 85® par la société BAYER, Aquamere H-1511® par la société HYDROMER; les sulfopolyesters vendus sous le nom de marque Eastman AQ® par la société Eastman Chemical Products, les dispersions vinyliques comme le Mexomère PAM® de la société CHIMEX et leurs mélanges, sont d'autres exemples de dispersion aqueuse de particules de polymères filmogènes hydrodispersibles.

Par « polymères amphiphiles ou associatifs » on entend des polymères comportant une au plusieurs partie hydrophiles qui les rendent partiellement solubles dans l'eau et une ou plusieurs parties hydrophobes par lesquelles les polymères s'associent ou interagissent. Les polymères associatifs suivants peuvent être utilisés : Nuvis FX1100 de Elementis, Aculyn 22, Aculyn 44, Aculyn 46 de Rohm&Haas, Viscophobe DB1000 de Amerchol. Les copolymères diblocs constitués d'un bloc hydrophile (polyacrylate, polyéthylène glycol) et d'un bloc hydrophobe (polystyrène, polysiloxane, peuvent également être utilisés.

La composition peut comporter une phase huileuse et le polymère filmogène peut être présent dans cette phase huileuse. Le polymère pourra alors être en dispersion ou en solution.

Comme exemples de dispersions non aqueuses de polymère filmogène lipodispersibles sous forme de dispersions non aqueuses de particules de polymère dans une ou plusieurs huiles de silicone et/ou hydrocarbonées et pouvant être stabilisées en leur surface par au moins un agent stabilisant, notamment un polymère séquencé, greffé ou statistique, on peut citer les dispersions acryliques dans l'isododécane comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .

Le polymère filmogène peut être choisi parmi les polymères et/ou copolymères blocs ou statistiques comportant notamment les polyuréthanes, polyacryliques, les silicones, les polymères fluorés, les gommes butyliques, les copolymères d'éthylènes, gommes naturelles et les alcools polyvinyliques et leurs mélanges.

Les polymères filmogènes vinyliques peuvent également résulter de l'homopolymérisation ou de la copolymérisation de monomères choisis parmi les esters vinyliques et les monomères styréniques.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styréniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

Selon un exemple de composition selon l'invention, le polymère filmogène peut être un polymère solubilisé dans une phase grasse liquide comprenant des huiles ou solvants organiques (on dit alors que le polymère filmogène est un polymère liposoluble). De préférence, la phase grasse liquide comprend une huile volatile, éventuellement en mélange avec une huile non volatile.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemple de polymères filmogènes liposolubles, on peut citer les copolymères d'ester vinylique et au moins un autre monomère qui peut être un ester vinylique, notamment le néodécanoate de vinyle, le benzoate de vinyle et le t-butyl benzoate de vinyle, une $\alpha$-oléfine, un alkylvinyléther, ou un ester allylique ou méthallylique.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2 232 303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Les compositions peuvent également comprendre au moins un polymère comportant au moins deux groupements susceptibles d'interagir par liaison hydrogène.

**Polymère comportant au moins deux groupements susceptibles d'interagir par des liaisons hydrogène**

Selon un mode de réalisation particulier, le polymère comprenant au moins deux groupes susceptibles d'interagir par liaison hydrogène est présent dans la composition en une teneur totale allant de 0,5 % à 50 % en poids par rapport au poids total de la composition, de préférence allant de 5 % à 50 % en poids, et mieux allant de 8 % à 45 % en poids, par exemple de 10 % à 40 % en poids, par rapport au poids total de ladite composition.

Selon l'invention, le polymère comprenant au moins deux groupes susceptibles d'interagir par liaison hydrogène peut appartenir aux deux familles suivantes :

1) des polymères comportant au moins deux groupes capables d'établir des interactions hydrogène, ces deux groupes étant situés dans la chaîne du polymère, et/ou
2) des polymères comportant au moins deux groupes capables d'établir des interactions hydrogène, ces deux groupes étant situés sur des greffons ou ramifications.

Par « polymère », on entend au sens de l'invention un composé ayant au moins 2 motifs de répétition, et de préférence au moins 3 motifs de répétition.

Par "motifs de répétition", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun de un à plusieurs hétéroatomes non pendants et se trouvant dans le squelette polymérique. Ces hétéroatomes sont choisis parmi les atomes d'azote, de soufre, de phosphore, de silicium et leurs associations, associés éventuellement à un ou plusieurs atome d'oxygène.

De façon préférée, ces groupes sont choisis parmi les groupes amide, sulfonamide, carbamate, thiocarbamate, urée, uréthane, thiourée, oxamido, guanidino, biguanidino et leurs combinaisons.

A titre d'exemple de polymère comprenant au moins deux groupes susceptibles d'interagir par liaison hydrogène, on peut citer :

- les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619 dont le contenu est incorporé à titre de référence ; en particulier les résines de polyamides (notamment comprenant des groupes alkyles ayant de 12 à 22 atomes de carbone) telles que celles décrites dans US-A-5,783,657 dont le contenu est incorporé à titre de référence,

- les résines de polyamides siliconées telles que décrites dans la demande EP-A-1 266 647, dans la demande de brevet français déposée sous le n° 0216039 dont le contenu est incorporé à titre de référence,
- les organopolysiloxanes comprenant au moins un groupe carboxyle, et de préférence les organopolysiloxanes comprenant au moins deux groupes carboxyles, par motif.

De tels polymères comprenant au moins deux groupes susceptibles d'interagir par liaison hydrogène sont notamment décrits dans les demandes EP-A-1 400 234, dont le contenu est incorporé à titre de référence et sont décrits de manière plus détaillée ci-après.

**Polymère siliconé**

Selon un premier mode de réalisation de l'invention, le polymère comprenant au moins deux groupes susceptibles d'interagir par liaison hydrogène est un polyamide siliconé.

Les polyamides siliconés sont de préférence solides à la température ambiante (25 °C) et pression atmosphérique (760 mm de Hg).

Les polyamides siliconés de la composition de l'invention peuvent être des polymères du type polyorganosiloxane comme par exemple ceux décrits dans les documents US-A-5,874,069, US-A-5,919,441, US-A-6,051,216 et

US-A-5,981,680.

Selon l'invention, les polymères siliconés peuvent appartenir aux deux familles suivantes :

(1) des polyorganosiloxanes comportant au moins deux groupes amides, ces deux groupes étant situés dans la chaîne du polymère, et/ou

(2) des polyorganosiloxanes comportant au moins deux groupes amides, ces deux groupes étant situés sur des greffons ou ramifications.

A) Selon une première variante, les polymères siliconés sont des polyorganosiloxanes tels que définis ci-dessus et dont les motifs amides sont disposés dans la chaîne du polymère.

Les polyamides siliconés peuvent plus particulièrement être des polymères comprenant au moins un motif répondant à la formule générale I :

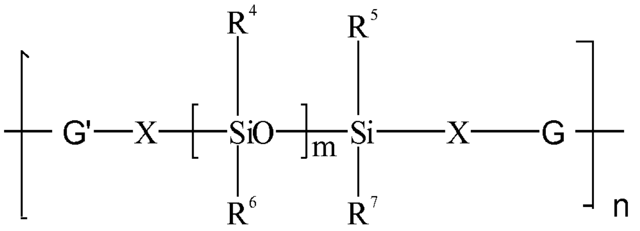

(I)

1) dans laquelle : G' représente C(O) quand G représente -C(O)-NH-Y-NH-, et G' représente -NH- quand G représente -NH-C(O)-Y-C(O)-

2) $R^4$, $R^5$, $R^6$ et $R^7$, identiques ou différents, représentent un groupe choisi parmi :

- les groupes hydrocarbonés, linéaires, ramifiés ou cycliques, en $C_1$ à $C_{40}$, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en $C_6$ à $C_{10}$, éventuellement substitués par un ou plusieurs groupes alkyle en $C_1$ à $C_4$,
- les chaînes polyorganosiloxanes contenant ou non un ou plusieurs atomes d'oxygène, de soufre et/ou d'azote,

3) les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote,

4) Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou

5) Y représente un groupe répondant à la formule :

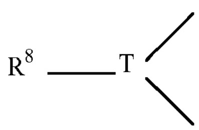

dans laquelle

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S, ou T représente un atome trivalent choisi parmi N, P et A1, et

- $R^8$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

6) n est un nombre entier allant de 2 à 500, de préférence de 2 à 200, et m est un nombre entier allant de 1 à 1000, de préférence de 1 à 700 et mieux encore de 6 à 200.

Selon un mode de réalisation de l'invention, 80 % des $R^4$, $R^5$, $R^6$ et $R^7$, du polymère sont choisis de préférence parmi les groupes méthyle, éthyle, phényle et 3,3,3-trifluoropropyle. Selon un autre mode, 80 % des $R^4$, $R^5$, $R^6$ et $R^7$, du polymère sont des groupes méthyle.
De préférence, Y représente un groupe choisi parmi :

a) les groupes alkylène linéaires en $C_1$ à $C_{20}$, de préférence en $C_1$ à $C_{10}$,
b) les groupes alkylène ramifiés pouvant comporter des cycles et des insaturations non conjuguées, en $C_{30}$ à $C_{56}$,
c) les groupes cycloalkylène en $C_5$-$C_6$,
d) les groupes phénylène éventuellement substitués par un ou plusieurs groupes alkyle en $C_1$ à $C_{40}$,
e) les groupes alkylène en $C_1$ à $C_{20}$, comportant de 1 à 5 groupes amides,
f) les groupes alkylène en $C_1$ à $C_{20}$, comportant un ou plusieurs substituants, choisis parmi les groupes hydroxyle, cycloalcane en $C_3$ à $C_8$, hydroxyalkyle en $C_1$ à $C_3$ et alkylamines en $C_1$ à $C_6$,
g) les chaînes polyorganosiloxane de formule :

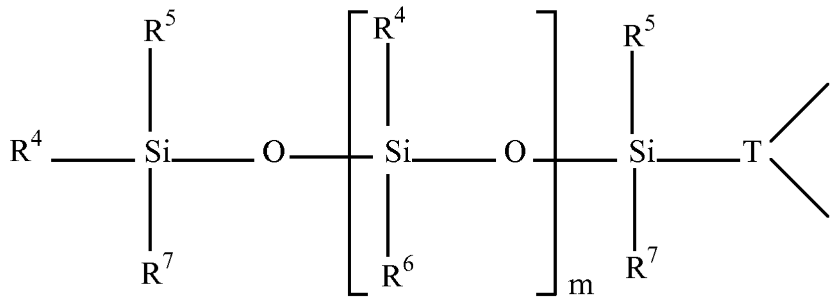

ou

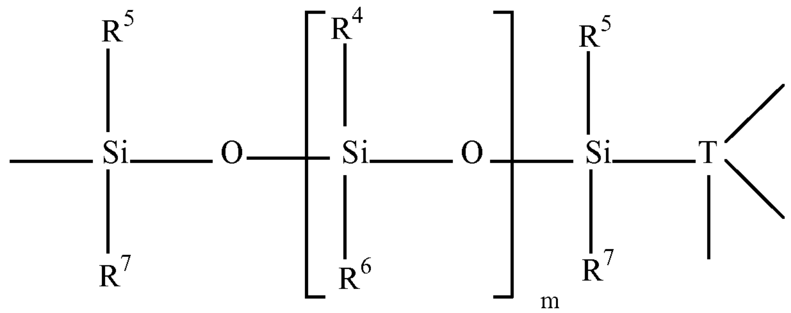

dans laquelle $R^4$, $R^5$, $R^6$, $R^7$, T et m sont tels que définis ci-dessus.

B) Selon la seconde variante, les polyamides siliconés peuvent être des polymères comprenant au moins un motif répondant à la formule (II) :

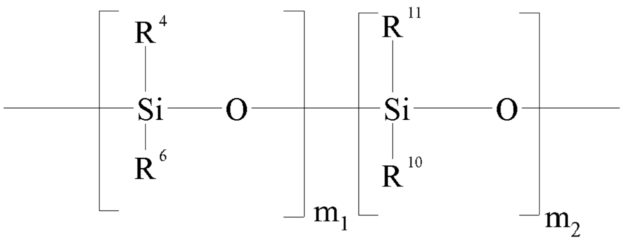

(II)

dans laquelle

- $R^4$ et $R^{6,}$ identiques ou différents, sont tels que définis ci-dessus pour la formule (I),
- $R^{10}$ représente un groupe tel que défini ci-dessus pour $R^4$ et $R^6$, ou représente le groupe de formule -X-G"-$R^{12}$ dans laquelle X sont tels que définis ci-dessus pour la formule (I) et $R^{12}$ représente un atome d'hydrogène ou un groupe hydrocarboné, linéaire, ramifié ou cyclique, saturé ou insaturé, en $C_1$ à $C_{50}$ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en $C_1$ à $C_4$,
  et G" représente -C(O)NH- et -HN-C(O)-.

$R^{11}$ représente le groupe de formule -X-G"-$R^{12}$ dans laquelle X, G" et $R^{12}$ sont tels que définis ci-dessus,

- $m_1$ est un nombre entier allant de 1 à 998, et
- $m_2$ est un nombre entier allant de 2 à 500.

Selon l'invention, le polymère siliconé peut être un homopolymère, c'est-à-dire un polymère comportant plusieurs motifs identiques, en particulier des motifs de formule (I) ou de formule (II).

Selon l'invention, on peut aussi utiliser un polymère constitué par un copolymère comportant plusieurs motifs de formule (I) différents, c'est-à-dire un polymère dans lequel l'un au moins des $R^4$, $R^5$, $R^6$, $R^7$, X, G, Y, m et n est différent dans l'un des motifs. Le copolymère peut être aussi formé de plusieurs motifs de formule (II), dans lequel l'un au moins des $R^4$, $R^6$, $R^{10}$, $R^{11}$, $m_1$ et $m_2$ est différent dans l'un au moins des motifs.

On peut encore utiliser un polymère comportant au moins un motif de formule (I) et au moins un motif de formule (II), les motifs de formule (I) et les motifs de formule (II) pouvant être identiques ou différents les uns des autres.

Selon une variante de l'invention, on peut encore utiliser un polyamide siliconé comprenant de plus au moins un motif hydrocarboné comportant deux groupes capables d'établir des interactions hydrogènes choisis parmi les groupes amide, sulfonamide, carbamate, thiocarbamate, urée, uréthane, thiourée, oxamido, guanidino, biguanidino et leurs combinaisons.

Ces copolymères peuvent être des polymères blocs, des polymères séquencés ou des polymères greffés.

Dans les formules (I) et (II), le groupe alkylène représentant X ou Y peut éventuellement contenir dans sa partie alkylène au moins l'un des éléments suivants:

1) 1 à 5 groupes amides, urée, uréthane, ou carbamate,
2) un groupe cycloalkyle en $C_5$ ou $C_6$, et
3) un groupe phénylène éventuellement substitué par 1 à 3 groupes alkyles identiques ou différents en $C_1$ à $C_3$.

Dans les formules (I) et (II), les groupes alkylènes peuvent aussi être substitués par au moins un élément choisi dans le groupe constitué de:

- un groupe hydroxy,
- un groupe cycloalkyle en $C_3$ à $C_8$,
- un à trois groupes alkyles en $C_1$ à $C_{40}$,
- un groupe phényle éventuellement substitué par un à trois groupes alkyles en $C_1$ à $C_3$,
- un groupe hydroxyalkyle en $C_1$ à $C_3$, et
- un groupe aminoalkyle en $C_1$ à $C_6$.

Dans ces formules (I) et (II), Y peut aussi représenter :

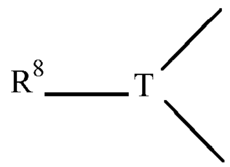

où $R^8$ représente une chaîne polyorganosiloxane, et T représente un groupe de formule :

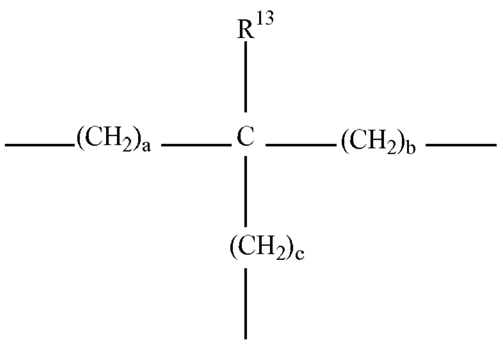

ou

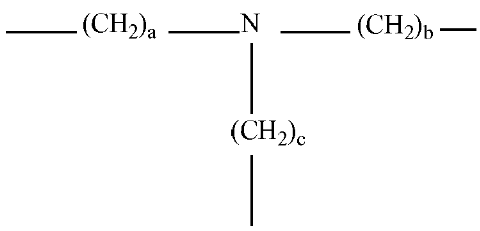

dans lesquelles a, b et c sont indépendamment des nombres entiers allant de 1 à 10, et $R^{13}$ est un atome d'hydrogène ou un groupe tel que ceux définis pour $R^4$, $R^5$, $R^6$ et $R^7$.

Dans les formules (I) et (II), $R^4$, $R^5$, $R^6$ et $R^7$ représentent de préférence, indépendamment, un groupe alkyle en $C_1$ à $C_{40}$, linéaire ou ramifié, de préférence un groupe $CH_3$, $C_2H_5$, n-$C_3H_7$ ou isopropyle, une chaîne polyorganosiloxane ou un groupe phényle éventuellement substitué par un à trois groupes méthyle ou éthyle.

Comme on l'a vu précédemment, le polymère peut comprendre des motifs de formule (I) ou (II) identiques ou différents.

Ainsi, le polymère peut être un polyamide contenant plusieurs motifs de formule (I) ou (II) de longueurs différentes, soit un polyamide répondant à la formule (III) :

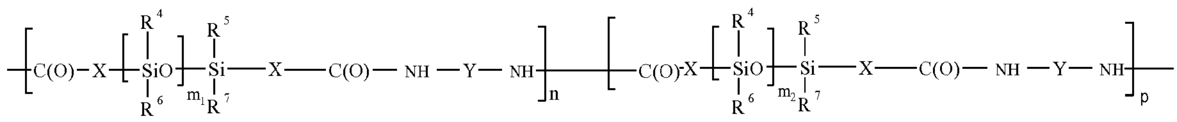

dans laquelle X, Y, n, $R^4$ à $R^7$ ont les significations données ci-dessus, $m_1$ et $m_2$ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le polymère peut répondre à la formule IV:

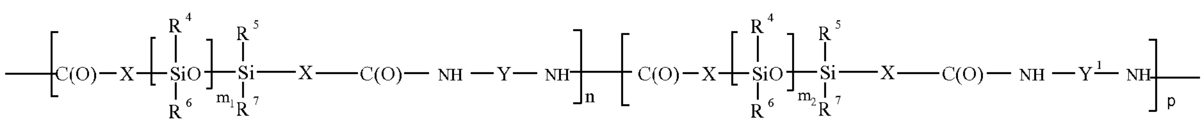

(IV)

dans laquelle $R^4$ à $R^7$, X, Y, $m_1$, $m_2$, n et p ont les significations données ci-dessus et $Y^1$ est différent de Y mais choisi parmi les groupes définis pour Y. Comme précédemment, les différents motifs peuvent être structurés pour former soit un copolymère bloc, soit un copolymère aléatoire, soit un copolymère alterné.

Dans ce premier mode de réalisation de l'invention, le polymère siliconé peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Selon l'invention, comme on l'a vu précédemment, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon une variante de réalisation de l'invention, on peut utiliser un copolymère de polyamide silicone et de polyamide hydrocarboné, soit un copolymère comportant des motifs de formule (I) ou (II) et des motifs polyamide hydrocarboné. Dans ce cas, les motifs polyamide-silicone peuvent être disposés aux extrémités du polyamide hydrocarboné.

Avantageusement, la composition comprend au moins un polymère polyamide/polydiméthylsiloxane, notamment un polymère de formule générale (I) possédant un indice m de valeur supérieure à 50, en particulier supérieure à 75, notamment compris entre 50 et 200, et par exemple d'environ 100.

De façon avantageuse, le polyamide siliconé de formule (I) a une masse moléculaire moyenne en poids allant de 10 000 à 500 000 g/mol.

De préférence encore, X et Y représentent indépendamment un groupe choisi parmi les groupes alkylène linéaires en $C_1$ à $C_{20}$, de préférence en $C_1$ à $C_{10}$.

A titre d'exemples de polymère utilisable, on peut citer un des polyamides siliconés, obtenus conformément aux exemples 1 à 3 du document US-A-5,981,680, tel que le produit commercialisé sous la référence DC 2-8179 par Dow Corning.

Selon une variante de réalisation de l'invention, le polymère est constitué par un homopolymère ou copolymère comportant des groupes uréthane ou urée. Ces polymères sont décrits en détail dans la demande WO 2003/106614.

La première composition peut contenir à la place du polyamide siliconé un polymère polyorganosiloxane contenant deux ou plusieurs groupes uréthanes et/ou urées, soit dans le squelette du polymère, soit sur des chaînes latérales ou comme groupes pendants.

Les polymères comportant au moins deux groupes uréthanes et/ou urées dans le squelette peuvent être des polymères comprenant au moins un motif répondant à la formule suivante :

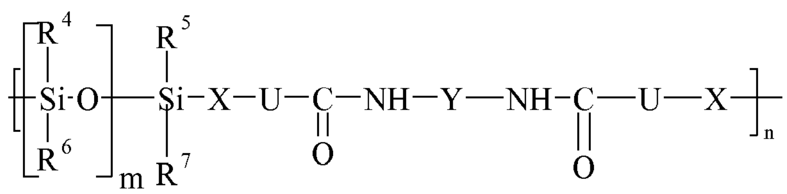

dans laquelle les $R^4$, $R^5$, $R^6$, $R^7$, X, Y, m et n ont les significations données ci-dessus pour la formule (I), et U représente -O- ou -NH-, afin que :

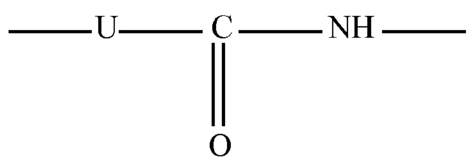

corresponde à un groupe uréthane ou urée.

Dans cette formule, Y peut être un groupe alkylène, en $C_1$ à $C_{40}$, linéaire ou ramifié, substitué éventuellement par un groupe alkyle en $C_1$ à $C_{15}$ ou un groupe aryle en $C_5$ à $C_{10}$. De préférence, on utilise un groupe $-(CH_2)_6-$.

Le polymère constituant le polymère siliconé peut être formé de motifs silicone uréthane et/ou silicone-urée de longueur et/ou de constitution différentes, et se présenter sous la forme de copolymères blocs, séquencés ou statistiques (aléatoires).

Comme dans le cas des polyamides silicones de formule (I), (II) ou (III), on peut utiliser dans l'invention des polyuréthanes ou des polyurées silicones ayant des motifs de longueur et de structure différentes, en particulier des motifs de longueurs différentes par le nombre d'unités silicones.

Les polymères et copolymères utilisés dans la composition de l'invention ont avantageusement une température de transition de l'état solide à l'état liquide allant de 45 °C à 190 °C. De préférence, ils présentent une température de transition de l'état solide à l'état liquide allant de 70 à 130 °C et mieux de 80 °C à 105 °C.

Le polyamide siliconé peut être présent dans la première composition en une teneur totale allant de 0,5 % à 70 % en poids par rapport au poids total de la composition, de préférence allant de 5 % à 50 % en poids, et mieux allant de 8 % à 45 % en poids, de préférence allant de 10 à 40 % en poids du poids total de ladite composition.

## Polymère hydrocarboné

Selon un second mode de réalisation de l'invention, le polymère comprenant au moins deux groupes susceptibles d'interagir par liaison hydrogène est un polymère masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619 dont le contenu est incorporé à titre de référence ; en particulier les résines de polyamides (notamment comprenant des groupes alkyles ayant de 12 à 22 atomes de carbone) telles que celles décrites dans US-A-5,783,657 dont le contenu est incorporé à titre de référence,

Le polymère selon l'invention est un solide non déformable à température ambiante (25°C).

Par "chaînes fonctionnalisées" au sens de l'invention, on entend une chaîne alkyle comportant un ou plusieurs groupes fonctionnels ou réactifs notamment choisis parmi les groupes hydroxyle, éther, oxyalkylène ou polyoxyalkylène, halogène, dont les groupes fluorés ou perfluorés, ester. En outre, les atomes d'hydrogène d'une ou plusieurs chaînes grasses peuvent être substitués au moins partiellement par des atomes de fluor.

De préférence, les motifs de répétition hydrocarbonée comportent au moins un atome d'azote en particulier non pendant. Ces motifs comportent, en outre, avantageusement, un groupe carbonyle.

Les motifs à hétéroatome sont en particulier des motifs amide formant un squelette du type polyamide, des motifs carbamate et/ou urée formant un squelette polyuréthane, polyurée et/ou polyurée-uréthane. De préférence, ces motifs sont des motifs amide. Avantageusement, les chaînes pendantes sont liées directement à l'un au moins des hétéroatomes du squelette polymérique.

Ce polymère peut comprendre entre les motifs hydrocarbonés des motifs oxyalkylénés.

En outre, ce polymère de la composition de l'invention comprend avantageusement de 40 à 98 % de chaînes grasses par rapport au nombre total des motifs à hétéroatome et des chaînes grasses et mieux de 50 à 95 %. La nature et la proportion des motifs à hétéroatome est fonction de la nature de la phase grasse et est en particulier similaire à la nature polaire de la phase grasse. Ainsi, plus les motifs à hétéroatome sont polaires et en proportion élevée dans ce polymère, ce qui correspond à la présence de plusieurs hétéroatomes, plus ce polymère a de l'affinité avec les huiles polaires. En revanche, plus les motifs à hétéroatome sont peu polaires voire apolaires ou en proportion faible, plus ce polymère a de l'affinité avec les huiles apolaires.

Ce polymère est avantageusement un polyamide. Aussi, l'invention a également pour objet une composition contenant, dans un milieu cosmétiquement acceptable, au moins un polymère de polyamide de masse moléculaire moyenne en poids inférieure à 100 000, comportant a) un squelette polymérique, ayant des motifs répétitifs amide, et b) éventuellement au moins une chaîne grasse pendante et/ou au moins une chaîne terminale éventuellement fonctionnalisées, ayant de 8 à 120 atomes de carbone et étant liées à ces motifs amide.

De préférence, les chaînes grasses pendantes sont liées à l'un au moins des atomes d'azote des motifs amide de ce polymère.

En particulier, les chaînes grasses de ce polyamide représentent de 40 à 98 % du nombre total des motifs amide et des chaînes grasses, et mieux de 50 à 95 %.

Avantageusement, ce polymère, et en particulier ce polyamide, de la composition selon l'invention présente une masse moléculaire moyenne en poids inférieure à 100 000 (notamment allant de 1000 à 100 000), en particulier inférieure à 50 000 (notamment allant de 1000 à 50 000), et plus particulièrement allant de 1000 à 30 000, de préférence de 2000 à 20 000, et mieux de 2000 à 10 000.

Ce polymère, et en particulier ce polyamide, est non soluble dans l'eau, notamment à 25 °C. En particulier, il ne comporte pas de groupe ionique.

En tant que polymères préférés utilisables dans l'invention, on peut citer les polyamides ramifiés par des chaînes grasses pendantes et/ou des chaînes grasses terminales ayant de 6 à 120 atomes de carbone et mieux de 8 à 120 et notamment de 12 à 68 atomes de carbone, chaque chaîne grasse terminale étant liée au squelette polyamide par au moins un groupe de liaison en particulier ester. De préférence, ces polymères comportent une chaîne grasse à

chaque extrémité du squelette polymérique et en particulier du squelette polyamide. Comme autre groupe de liaison on peut citer les groupes éther, amine, urée, uréthane, thioester, thiurée, thiouréthane.

Ces polymères sont de préférence des polymères résultant d'une polycondensation entre un diacide carboxylique ayant au moins 32 atomes de carbone

(ayant notamment de 32 à 44 atomes de carbone) avec une amine choisie parmi les diamines ayant au moins 2 atomes de carbone (notamment de 2 à 36 atomes de carbone) et les triamines ayant au moins 2 atomes de carbone (notamment de 2 à 36 atomes de carbone). Le diacide est de préférence un dimère issu d'acide gras à insaturation éthylénique ayant au moins 16 atomes de carbone, de préférence de 16 à 24 atomes de carbone, comme l'acide oléique, linoléique ou linolénique. La diamine est de préférence l'éthylène diamine, l'hexylène diamine, l'hexaméthylène diamine. La triamine est par exemple l'éthylène triamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Ces polymères sont plus spécialement ceux décrits dans le document US-A-5,783,657 de la société Union Camp. Chacun de ces polymères satisfait notamment à la formule (I) suivante :

(I) 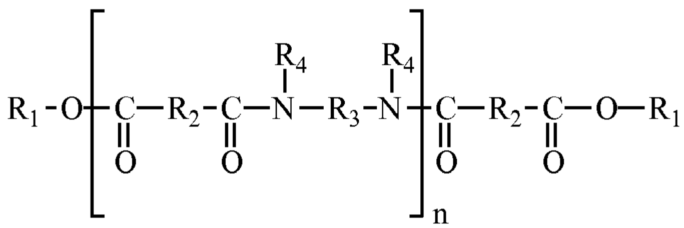

dans laquelle n désigne un nombre entier de motifs amide tel que le nombre de groupes ester représente de 10 % à 50 % du nombre total des groupes ester et amide; $R_1$ est à chaque occurrence indépendamment un groupe alkyle ou alcényle ayant au moins 4 atomes de carbone et notamment de 4 à 24 atomes de carbone ; $R_2$ représente à chaque occurrence indépendamment un groupe hydrocarboné en $C_4$ à $C_{42}$ à condition que 50 % des groupes $R_2$ représentent un groupe hydrocarboné en $C_{30}$ à $C_{42}$ ; $R_3$ représente à chaque occurrence indépendamment un groupe organique pourvu d'au moins 2 atomes de carbone, d'atomes d'hydrogène et optionnellement d'un ou plusieurs atomes d'oxygène ou d'azote ; et $R_4$ représente à chaque occurrence indépendamment un atome d'hydrogène, un groupe alkyle en $C_1$ à $C_{10}$ ou une liaison directe à $R_3$ ou à un autre $R_4$ de sorte que l'atome d'azote auquel sont liés à la fois $R_3$ et $R_4$ fasse partie d'une structure hétérocyclique définie par $R_4$-N-R3, avec au moins 50 % des $R_4$ représentant un atome d'hydrogène.

Dans le cas particulier de la formule (I), les chaînes grasses terminales éventuellement fonctionnalisées au sens de l'invention sont des chaînes terminales liées au dernier hétéroatome, ici l'azote, du squelette polyamide.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5 et mieux supérieur à 2. De préférence, $R_1$ est un groupe alkyle en $C_{12}$ à $C_{22}$ et de préférence en $C_{16}$ à $C_{22}$. Avantageusement, $R_2$ peut être un groupe hydrocarboné (alkylène) en $C_{10}$ à $C_{42}$. De préférence, 50 % au moins et mieux au moins 75 % des $R_2$ sont des groupes ayant de 30 à 42 atomes de carbone. Les autres $R_2$ sont des groupes hydrogénés en $C_4$ à $C_{19}$ et même en $C_4$ à $C_{12}$. De préférence, $R_3$ représente un groupe hydrocarboné en $C_2$ à $C_{36}$ ou un groupe polyoxyalkyléné et $R_4$ représente un atome d'hydrogène. De préférence, $R_3$ représente un groupe hydrocarboné en $C_2$ à $C_{12}$.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

En général, les polymères de formule (I) se présentent sous forme de mélanges de polymères, ces mélanges pouvant en outre contenir un produit de synthèse correspondant à un composé de formule (I) où n vaut 0, c'est-à-dire un diester.

A titre d'exemple de polymères comprenant au moins deux groupes susceptibles d'interagir par liaison hydrogène utilisables dans les compositions selon l'invention, on peut citer les produits commerciaux vendus par la société Arizona Chemical sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94 °C. Ces produits commerciaux sont un mélange de copolymères d'un diacide en C36 condensé sur l'éthylène diamine, de masse moléculaire moyenne en poids d'environ 6000. Les groupes ester terminaux résultent de l'estérification des terminaisons d'acide restantes par l'alcool cétylique, stéarylique ou leurs mélanges (appelés aussi alcool cétylstéarylique).

Comme polymère comprenant au moins deux groupes susceptibles d'interagir par liaison hydrogène utilisable

dans les compositions selon l'invention, on peut encore citer les résines polyamides résultant de la condensation d'un acide di-carboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US-A-3,645,705 et US-A-3,148,125. Plus spécialement, on utilise les Versamid® 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5,500,209.

Il est aussi possible d'utiliser des résines de polyamides comme celles décrites dans les brevets US-A-5,783,657 et US-A-5,998,570.

Le polymère présent dans la composition selon l'invention a avantageusement une température de ramollissement supérieure à 65 °C et pouvant aller jusqu'à 190 °C. De préférence, il présente une température de ramollissement allant de 70 à 130 °C et mieux de 80 à 105 °C.

## Matières colorantes

Les compositions selon l'invention peuvent avantageusement contenir un agent de coloration qui peut être choisi parmi les colorants hydrosolubles ou liposolubles, les pigments, les nacres et leurs mélanges.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids, et en particulier de 0,05 à 25 % en poids par rapport au poids total de la composition.

Par pigments, il faut comprendre des particules blanches ou colorées, minérales ou organiques, insolubles dans une solution aqueuse, destinées à colorer et/ou opacifier le film résultant.

Les pigments peuvent être présents à raison de 0,01 à 20 % en poids, notamment de 0,01 à 15 % en poids, et en particulier de 0,02 à 10 % en poids, par rapport au poids total de la composition cosmétique.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé,

brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Par « colorants », il faut comprendre des composés généralement organiques solubles dans les corps gras comme les huiles ou dans une phase hydroalcoolique.

Les colorants liposolubles peuvent être choisis parmi le rouge Soudan, le DC Red 17, le DC Green 6, le β-carotène, le brun Soudan, le DC Yellow 11, le DC Violet 2, le DC orange 5, le jaune quinoléine. Les colorants hydrosolubles sont par exemple le jus de betterave, le bleu de méthylène.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets.

Les particules à reflet métallique utilisables dans l'invention sont en particulier choisies parmi :

- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, A1, Ni, Sn, Mg, Cr, Mo, Ti, Zr, Pt, Va, Rb, W, Zn, Ge, Te, Se et leurs mélanges ou alliages. Ag, Au, Cu, A1, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

Il peut encore s'agir de particules comportant un substrat de verre comme celles commercialisées par la société

NIPPON SHEET GLASS sous les dénominations MICROGLASS METASHINE.

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : $A1/SiO_2/A1/SiO_2/A1$, des pigments ayant cette structure étant commercialisés par la société DUPONT DE NEMOURS ; $Cr/MgF_2/Al/MgF_2/Cr$, des pigments ayant cette structure étant commercialisés sous la dénomination CHROMAFLAIR par la société FLEX ; $MoS_2/SiO_2/A1/SiO_2/MoS_2$ $Fe_2O_3/SiO_2/A1/SiO_2/Fe_2O_3$, et $Fe_2O_3/SiO_2/Fe_2O_3/SiO_2/Fe_2O_3$, des pigments ayant ces structures étant commercialisés sous la dénomination SICOPEARL par la société BASF ; $MoS_2/SiO_2$/mica-oxyde/$SiO_2/MoS_2$ ; $Fe_2O_3/SiO_2$/mica-oxyde/$SiO_2/Fe_2O_3$ ; $TiO_2/SiO_2/TiO_2$ et $TiO_2/A1_2O_3/TiP_2$ ; $SnO/TiO_2/SiO_2/TiO_2/$ SnO , $Fe_2O_3/SiO_2/Fe_2O_3$ ; SnO/mica/$TiO_2/SiO_2/TiO_2$/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure $Fe_2O_3/SiO_2/A1/ SiO_2/Fe_2O_3$ on passe du doré-vert au gris-rouge pour des couches de $SiO_2$ de 320 à 350 nm ; du rouge au doré pour des couches de $SiO_2$ de 380 à 400 nm ; du violet au vert pour des couches de $SiO_2$ de 410 à 420 nm ; du cuivre au rouge pour des couches de $SiO_2$ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE® HC par la société WACKER.

## Charge

Une composition selon l'invention peut comprendre une charge, notamment en une teneur totale allant de 0,01 à 30 %, en particulier de 0,01 % à 20 % en poids, par exemple allant de 0, 1 % à 15 % ou de 0,5 % à 10 % en poids par rapport au poids total de la composition.

Par « charge », il faut comprendre au sens de la présente invention, des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téfloe), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Il peut également s'agir de particules comprenant un copolymère, ledit copolymère comprenant du triméthylol hexyllactone. En particulier, il peut s'agir d'un copolymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI.

## Ingrédients cosmétiques usuels additionnels

La composition selon l'invention peut comprendre en outre tout ingrédient cosmétique usuel pouvant être choisi notamment parmi les antioxydants, les parfums, les conservateurs, les neutralisants, les tensioactifs, les filtres solaires, les édulcorants, les vitamines, les hydratants, les émollients, les actifs hydrophiles ou lipophiles, les agents anti-radicaux libres, les séquestrants, et leurs mélanges.

Bien entendu, l'homme du métier veillera à choisir les éventuels ingrédients complémentaires et/ou leur quantité de telle manière que les propriétés avantageuses de la composition selon l'invention ne soient pas ou substantiellement

pas altérées par l'adjonction envisagée.

L'homme du métier pourra choisir la forme galénique appropriée, ainsi que sa méthode de préparation, sur la base de ses connaissances générales, en tenant compte, d'une part, de la nature des constituants utilisés, notamment de leur solubilité dans le support, et d'autre part, de l'application envisagée pour la composition.

De façon préférée, la composition selon l'invention comprend moins de 3%, ou mieux, moins de 1% d'eau en poids par rapport au poids total de la composition. De façon encore préférée la composition est totalement anhydre. Par anhydre, on entend notamment que l'eau n'est de préférence pas ajoutée délibérément dans la composition mais peut être présente à l'état de trace dans les différents composés utilisés dans la composition.

La composition selon l'invention peut être destinée au soin et/ou au maquillage des matières kératiniques, notamment des lèvres et de la peau, en particulier des lèvres.

Les exemples qui suivent illustrent de manière non limitative l'invention.

Les quantités sont exprimées en pourcentage pondéral.

## Dispositifs d'application

On va maintenant décrire des exemples de dispositifs permettant, entre autres, de mettre en oeuvre un procédé de traitement cosmétique comportant les étapes consistant à:

a) réchauffer une surface d'application d'une masse de produit solide, à l'aide d'une source artificielle de chaleur située à l'extérieur de la masse de produit, notamment une surface d'application d'un bâtonnet de produit, pour la porter à une température supérieure à celle d'une portion de la masse de produit éloignée de la surface d'application et qui reste solide lors de l'application, et

b) appliquer la surface d'application ainsi réchauffée sur une région à traiter, notamment la peau ou les lèvres.

La description de ces dispositifs est faite en se référant au dessin annexé, sur lequel :

- la figure 1 représente, de manière schématique, en élévation, un exemple de dispositif de conditionnement et d'application réalisé conformément à l'invention,
- la figure 2 représente isolément, avec coupe longitudinale partielle et schématique, le capot du dispositif de la figure 1,
- la figure 3 illustre, de manière schématique et partielle, le réchauffement de l'extrémité du bâtonnet par contact avec une surface chaude,
- la figure 4 représente de manière schématique et partielle un exemple de réalisation de l'organe chauffant,
- les figures 5 à 7 illustrent des détails de réalisation de variantes d'organes chauffants,
- la figure 8 représente, de manière schématique, une variante de réalisation du dispositif de conditionnement et d'application,
- la figure 9 est une coupe schématique et partielle du dispositif de la figure 8, après mise en place dans le logement correspondant du boîtier,
- la figure 10 représente un bâtonnet et des moyens de support associés,
- la figure 11 représente en élévation une variante de réalisation du dispositif de conditionnent et d'application,
- la figure 12 est une coupe longitudinale, partielle et schématique, du dispositif de la figure 11,
- la figure 13 est une coupe longitudinale, partielle et schématique, d'une variante de réalisation du dispositif,
- la figure 14 représente une variante de conditionnement du produit, et
- la figure 15 précédemment décrite, illustre la mesure du coefficient de frottement dynamique.

Le dispositif 1 de conditionnement et d'application représenté à la figure 1 comporte une partie de base 2 qui supporte une masse de produit selon l'invention se présentant sous la forme d'un bâtonnet S de produit, et un capot 3 qui peut se fixer sur la partie de base 2 pour fermer le dispositif 1 en l'absence d'utilisation.

La partie de base 2 peut être de tout type connu permettant de déplacer le bâtonnet S au fur et à mesure de sa consommation.

La partie de base 2 comporte par exemple deux parties 5 et 6 pouvant tourner l'une par rapport à l'autre, et un mécanisme permettant de transformer la rotation relative des deux parties 5 et 6 en un déplacement axial selon l'axe longitudinal X du bâtonnet S.

Le bâtonnet S est par exemple porté, au sein de ce mécanisme, par une cupule 58 telle que représentée à la figure 10, comportant des ergots 59 engagés dans deux pièces appartenant respectivement aux parties 5 et 6, dont l'une comporte des fentes rectilignes longitudinales et l'autre des fentes hélicoïdales, de telle sorte qu'une rotation de ces deux pièces s'accompagne d'un déplacement axial de la cupule et du bâtonnet S.

Des exemples de mécanismes pouvant convenir sont décrits dans les publications US 6,340,258, US 6,086,276, US 6,371,673, US 5,171,096 et US 7,293,926, dont le contenu est incorporé par référence.

Le capot 3 comporte un dispositif de chauffage 10 qui permet de réchauffer l'extrémité 11 du bâtonnet S préalablement à son application sur les matières kératiniques, par exemple la peau ou les lèvres.

Le dispositif de chauffage 10 peut loger une source électrique non apparente, par exemple contenant une ou plusieurs piles ou accumulateurs, et un organe de chauffage comportant par exemple une résistance électrique alimentée par la source électrique.

Des exemples d'organes de chauffage susceptibles de convenir sont divulgués dans US 2007/0,286,665 A1, par exemple.

L'organe de chauffage est disposé de manière à élever la température d'une surface de chauffage 13 qui, dans l'exemple des figures 1 et 2, peut venir au contact du bâtonnet S, comme illustré sur la figure 3, afin d'élever la température de l'extrémité 11 distale de celui-ci.

Le dispositif de chauffage 10 peut comporter un interrupteur 14 permettant à l'utilisateur de mettre en marche ou d'arrêter le dispositif de chauffage 10, ainsi qu'un témoin 15 de fonctionnement, par exemple un voyant lumineux qui s'allume lorsque la surface de chauffage 13 est en cours de chauffage.

Le dispositif de chauffage 10 peut éventuellement comporter tout moyen de régulation de la température de la surface de chauffage 13, afin que celle-ci n'excède pas une valeur prédéfinie.

Lorsque la surface de chauffage est inaccessible à l'utilisateur, une température de chauffage plus élevée mais compatible avec le produit peut être acceptée. Par contre, lorsque la surface de chauffage 13 peut être contactée par l'utilisateur, une température n'excédant pas 65 °C est préférée.

Le dispositif de chauffage 10 peut également, le cas échéant, comporter une temporisation qui permet de ne réchauffer l'extrémité 11 du bâtonnet S que pendant une durée prédéfinie, afin d'éviter une usure prématurée de la source d'énergie électrique et/ou éviter de porter l'ensemble du bâtonnet à une température excessive.

Le dispositif de chauffage 10 peut avantageusement comporter tout capteur adapté permettant de ne déclencher le fonctionnement du chauffage qu'en cas de contact effectif de la surface de chauffage avec l'extrémité 11 du bâtonnet S.

Par exemple, le dispositif de chauffage 10 peut comporter un capteur de pression de contact entre la surface de chauffage 13 et le bâtonnet S, et n'autoriser le chauffage de la surface de chauffage 13 qu'en cas de contact avéré avec le bâtonnet S.

La surface de chauffage 13 peut être définie par exemple par une pièce de contact 20, par exemple mobile axialement selon l'axe X relativement au corps 22 du dispositif de chauffage 10 contre l'action de rappel d'un organe de rappel élastique 23, tel que par exemple un ressort logé à l'intérieur de la pièce de contact 20, comme illustré à la figure 4.

Sur cette figure 4, on a représenté un dispositif de chauffage comportant une résistance électrique 25 plaquée dans le fond de la pièce de contact 20, de façon à être au plus près de la surface de chauffage 13.

La pièce de contact 20 peut par exemple comprendre un métal bon conducteur de la chaleur, avec une faible épaisseur de paroi, de façon à présenter une faible inertie thermique. Dans certains modes de réalisation, la pièce de contact 20 peut par exemple comprendre de l'aluminium.

On peut donner à la surface de chauffage 13 toute forme adaptée à la géométrie de l'extrémité 11 du bâtonnet, par exemple une forme en biseau sensiblement complémentaire de la forme de l'extrémité 11 du bâtonnet S, comme illustré sur les figures 1 et 2, ou une forme autre, par exemple une forme concave vers le bâtonnet S, notamment une forme de calotte sphérique comme illustré sur la figure 5, une forme conique ou tronconique comme illustré à la figure 6 ou une forme sensiblement plane et perpendiculaire à l'axe X, comme illustré à la figure 7.

Lorsque la forme de la surface de chauffage 13 est non symétrique de révolution autour de l'axe X, le dispositif 1 peut comporter des moyens d'indexation en rotation de la partie de base 2 et du capot 3, de façon à ne permettre la fixation du capot 3 sur la partie de base 2 que dans une orientation angulaire prédéfinie entre les deux, dans laquelle la surface de chauffage 13 peut venir s'appliquer d'une manière prédéfinie, compatible avec sa géométrie, contre le bâtonnet S.

Le bâtonnet S, qui est par exemple un bâtonnet de rouge à lèvres, peut avoir une section comprise entre 0.1 et 5 $cm^2$, voire entre 0,15 et 1 $cm^2$, et le dispositif 1 peut s'utiliser en allumant d'abord le dispositif de chauffage 10 puis en attendant la durée nécessaire pour que l'extrémité 11 du bâtonnet qui définit la surface d'application soit portée à la température recherchée.

La mise à température peut par exemple être signalée par le voyant 15, lequel peut par exemple passer d'un état d'allumage continu signalant la mise en marche du dispositif à un allumage clignotant ou changer de couleur lorsque la température est atteinte. D'autres méthodes pour indiquer l'état d'allumage peuvent être employées sans toutefois s'écarter de la portée de l'invention.

Une fois l'extrémité du bâtonnet réchauffée, la partie de base 2 peut être séparée du capot 3 et l'utilisateur peut appliquer le produit du bâtonnet sur les lèvres ou d'autres matières kératiniques. Le ramollissement du produit à l'extrémité 11 du bâtonnet assure une application confortable, un bon transfert sur les lèvres avec un dépôt épais et éventuellement brillant à l'application.

Par exemple, l'application se fait sans utiliser d'applicateur. Autrement dit, seule la composition, et plus précisément la surface ramollie est mise en contact direct avec la région à traiter.

Le corps du bâtonnet S est à la température ambiante ou à une température légèrement supérieure, mais insuffisante pour compromettre la résistance mécanique nécessaire pour supporter les efforts mécaniques engendrés par l'application. L'écart de température entre la surface d'application et le corps du bâtonnet, notamment à l'extrémité opposée à la surface d'application, et par exemple d'au moins 20 °C, voire d'au moins 30 °C lorsque le bâtonnet présente sa longueur initiale, à la première utilisation.

Le dispositif 1 peut s'utiliser d'une manière similaire pour maquiller la peau, le bâtonnet pouvant alors être de section plus grande, le cas échéant.

Le dispositif de chauffage peut ne pas être incorporé à un capot 3 du dispositif de conditionnement, mais être présent dans un boîtier 40 distinct du dispositif de conditionnement du bâtonnet S, comme illustré aux figures 8 et 9.

Le boîtier 40 peut loger une source électrique et/ou comporter un moyen de raccordement à une source électrique, par exemple le secteur *via* un transformateur basse tension.

Le boîtier 40 peut comporter également des moyens de mise en marche 41, tels que par exemple un interrupteur marche/arrêt, ainsi qu'un ou plusieurs voyants 42 et 55 pour signaler la mise sous tension et/ou la fin de la mise en température.

Dans l'exemple des figures 8 et 9, le boîtier 40 comporte une ouverture 46 dans laquelle la partie de base 2 peut être introduite au moins partiellement, comme illustré sur la figure 9, afin d'amener l'extrémité 11 du bâtonnet à proximité d'un moyen de chauffage 50 présent dans le boîtier 40.

L'ouverture 46 est par exemple de section adaptée à l'une des pièces de la partie de base, de façon à ce que l'engagement de la partie de base dans le boîtier amène l'extrémité 11 du bâtonnet dans une position prédéfinie, au moins selon deux directions de l'espace, relativement au moyen de chauffage.

Le boîtier 40 peut comporter tout capteur adapté 51 permettant de détecter la mise en place de la partie de base 2 sur le boîtier 40 et éventuellement le positionnement du bâtonnet relativement au moyen de chauffage.

Le chauffage de l'extrémité du bâtonnet S peut avoir lieu par conduction, au contact d'une surface chaude, à l'instar de ce qui a été décrit ci-dessus. Dans ce cas, le moyen de chauffage comporte une surface de chauffage qui peut être portée à la température adéquate par tout moyen de chauffage, par exemple une résistance électrique.

Le chauffage de l'extrémité du bâtonnet peut encore s'effectuer sans contact, par exemple par rayonnement IR et/ou convection, et/ou par vibrations et/ou rayonnement radio-électrique,, ou toute autre source procurant de la chaleur.

Comme évoqué plus haut, le boîtier 40 peut comporter tout capteur adapté, notamment optique, capable d'évaluer la distance entre l'extrémité 11 du bâtonnet et le moyen de chauffage 50, afin de ne permettre la mise en marche de celui-ci que lorsqu'une distance prédéfinie est respectée et/ou afin de régler la puissance de chauffage en fonction de l'éloignement entre le moyen de chauffage et l'extrémité du bâtonnet S.

Dans certaines variantes, le moyen de chauffage 50 peut être un système de chauffage par émission d'un rayonnement infrarouge vers l'extrémité 11 du bâtonnet, par exemple au moyen d'une lampe halogène ou incandescente, ou par soufflage d'air chaud vers l'extrémité 11.

Dans certaines variantes, l'extrémité 11 du bâtonnet S peut encore être réchauffée par exposition à un rayonnement radioélectrique, par exemple micro-ondes, focalisé à l'extrémité 11 du bâtonnet S.

Dans encore d'autres variantes, l'extrémité 11 du bâtonnet S peut être réchauffée par des vibrations ultrasonores.

Dans la variante de réalisation des figures 11 et 12, le dispositif de chauffage 60 comporte un moyen de chauffage 62 qui est solidaire de la partie de base 2 et qui peut comporter, comme illustré, un organe de chauffage 62 de forme annulaire, qui peut être traversé par le bâtonnet S. L'organe de chauffage 62 est par exemple de section supérieure ou égale à celle du bâtonnet S.

Le dispositif de chauffage 60 peut comporter par exemple un organe de commande 64 sur lequel l'utilisateur peut appuyer pour déclencher le fonctionnement de l'organe de chauffage 62. L'organe de chauffage 62 peut comporter par exemple une résistance chauffante qui permet de réchauffer par conduction, convection et/ou rayonnement (par exemple infrarouge, micro-ondes...) l'extrémité 11 du bâtonnet S.

Le cas échéant, l'organe de chauffage 62 peut également participer à l'application du produit associé au bâtonnet S et peut à cet effet présenter une face supérieure 70 de forme adaptée, par exemple biseautée.

Pour utiliser le dispositif dans l'exemple considéré, l'utilisateur peut amener l'extrémité 11 du bâtonnet au niveau de l'organe de chauffage 62 et déclencher le chauffage en appuyant sur l'organe de commande 64.

Le dispositif de chauffage peut comporter un indicateur lumineux 72 signalant à l'utilisateur le fonctionnement de l'organe de chauffage 62.

L'utilisateur peut interrompre ensuite le chauffage lorsqu'il constate visuellement que l'extrémité 11 du bâtonnet a changé d'aspect suite à l'élévation de température, par exemple est devenue brillante.

L'utilisateur peut alors éventuellement, à ce moment, déplacer un peu plus vers le haut l'extrémité 11 de façon à faciliter l'application du produit, sans contact avec l'organe chauffant 62. En variante, l'utilisateur peut procéder à

l'application du produit avec un contact non seulement du bâtonnet S mais également de l'organe chauffant 62 sur les lèvres ou la peau.

Le cas échéant, la surface de l'organe de chauffage 62 susceptible de contacter la peau peut être floquée ou présenter un aspect de surface texturé facilitant l'application.

Dans la variante illustrée à la figure 13, le bâtonnet S passe au travers d'un organe de chauffage 62 définissant une ouverture 76 de section rétrécie par rapport à la section du corps du bâtonnet.

Le ramollissement du bâtonnet S au contact de l'organe de chauffage 62 peut ainsi s'accompagner, dans cet exemple, d'une déformation du bâtonnet au travers de l'organe de chauffage 62. Cela peut accroître la précision d'application du produit et éviter que le bâtonnet S ne puisse être avancé relativement à l'organe de chauffage 62 tant qu'un ramollissement suffisant n'est pas atteint.

La surface extérieure de l'organe de chauffage 62 peut être effilée, comme illustré à la figure 13, afin de réduire la surface de contact entre la région traitée et l'organe de chauffage 62.

La figure 14 représente une variante de réalisation où la masse de produit S associée au bâtonnet S est supportée par une tige 200, et convient par exemple à un usage unique.

La surface d'application 202 est réchauffée en étant par exemple amenée au contact ou à proximité d'une surface chaude, par exemple en l'introduisant dans un boîtier pourvu d'un moyen de chauffage tel que le boîtier précédemment décrit en référence aux figures 8 et 9.

Dans le présent texte, les teneurs, sauf mention express contraire, sont exprimées en poids par rapport au poids total de la composition.

L'invention est illustrée plus en détails par les exemples décrits ci-après donnés à titre illustratif et sans caractère limitatif. Les pourcentages sont des pourcentages en poids. Dans les exemples qui suivent, les pourcentages en poids sont indiqués par rapport au poids total de la composition.

**EXEMPLES:**

**Exemple 1 : Composition de rouge à lèvres**

On a préparé un rouge à lèvres selon l'invention ayant la composition suivante :

| Phase | Ingrédient | Fonction | Composition 1(% en poids) |
|---|---|---|---|
| A | OCTYLDODECANOL | CORPS GRAS | 5 |
| | DIISOSTEARYL MALATE | CORPS GRAS | 10 |
| B | POLYBUTENE (Indopol H 100 d'INEOS) | POLYMERE | 10 |
| | POLYESTER : Pentaérythritol 20 / acide benzoïque 4 / acide isostéarique 56 / acide isophtalique 20 (tel que préparé dans l'exemple 2 de EP-A-1870082) | POLYMERE | 20 |
| C | BIS-BEHENYL/ISOSTEARYL/PHYTOSTERYL DIMER DILINOLEYL DIMER DILINOLEATE (Plandool G de Nippon Fine Chemical) | CORPS GRAS | 10 |
| | BIS-DIGLYCERYL POLYACYLADIPATE-2 (Softisan 649 de Sasol) | CORPS GRAS | 14,72 |
| | POLYESTER D'ACIDE DIMER C36 ET D'HUILE DE RICIN HYDROGÉNÉE(Risocast de Kokyu Alcohol Kogyo) | CORPS GRAS | 10 |
| D | POLYETHYLENE (Performalene 400 de New pahse technologies) | CORPS GRAS | 10 |
| E | BLUE 1 LAKE | COLORANT | 1,2 |
| | OXYDE DE TITANE | COLORANT | 2,63 |
| | RED 28 LAKE | COLORANT | 1,65 |
| | OXYDE DE FER | COLORANT | 4,8 |
| 1 | Total | | 100 |

Protocole :

Dans un premier temps on disperse les pigments de la phase E dans une partie de la phase A.

On mélange le reste des ingrédients liposolubles de la phase B et C et les cires de la phase D à une température de 100 °C puis on y ajoute le broyat et le reste de la phase A. On mélange le tout à une température de 100 °C jusqu'à l'obtention d'un mélange bien homogène.

Enfin on coule la composition dans un moule pour lui donner la forme d'un stick de diamètre 11.06 mm. On place ensuite le moule dans un congélateur à -18 °C.

La dureté du stick à 20°C est de 124 $Nm^{-1}$.

*Evaluation :*

A température ambiante (20°C), la composition ainsi obtenue, lorsqu'elle est appliquée sur les lèvres, dépose très peu et est très accrocheuse (glisse mal sur les lèvres et se délite difficilement).

En revanche, après chauffage de l'extrémité du stick (en général en forme de biseau), à 60°C pendant 10 secondes, il est possible d'appliquer facilement (avec un bon glissant et un délitage aisé) le stick sur les lèvres et d'obtenir un dépôt de maquillage épais.

De plus, le maquillage déposé sur les lèvres avec la composition 1 préalablement chauffée présente une tenue de la couleur améliorée, ainsi qu'une brillance améliorée par rapport au dépôt de maquillage obtenu avec la même composition 1 non chauffée appliquée à température ambiante (20°C).

**Exemple 2 : Composition de rouge à lèvres**

On a préparé un rouge à lèvres selon l'invention ayant la composition suivante :

| Phase | Ingrédients | Fonction | Composition 2 selon l'invention (% en poids) |
|---|---|---|---|
| A | OCTYLDODECYL PPG3 MYRISTYL ETHER DIMER DILINOLEATE (Liquiwax polyEFA OR de Arch Personal care) | CORPS GRAS | 2,28 |
| | TRI-MELLITATE DE TRI-DECYLE | CORPS GRAS | 2,28 |
| B | Trimethyl 1,1,3,5,5-pentaphenyl trisiloxane (PH-1555 HRI de Dow Corning ) | SILICONE | 50,36 |
| C | POLYESTER: Pentaérythritol 20 / acide benzoïque 4 / acide isostéarique 56 / acide isophtalique 20 (tel que préparé dans l'exemple 2 de EP-A-1870082) | POLYMERE | 20 |
| | | | |
| D | HYDROGENATED COCOGLYCERIDES (Softisan 100 de SASOL) | CORPS GRAS | 2 |
| | COPOLYMERE ACETATE DE VINYLE / STEARATE D'ALLYLE (65/35) (mexomere PQ de Chimex) | POLYMERE | 3,95 |
| | POLYLAURATE DE VINYLE (Mexomere PP de Chimex) | POLYMERE | 5,92 |
| | POLY ACRYLATE DE STEARYLE (Intelimer IPA 13-1 d'Air products and chemicals) | POLYMERE | 2,96 |
| E | HUILE DE JOJOBA HYDROGENEE (Jojoba Wax flakes de Desert Whale) | CORPS GRAS | 2 |

(suite)

| Phase | Ingrédients | Fonction | Composition 2 selon l'invention (% en poids) |
|---|---|---|---|
| | CIRE MICROCRISTALLINE (Base Wax 30540 de paramelt) | CORPS GRAS | 3 |
| | C30-50 ALCOHOLS (Performacol 550-L Alcohol de New phase technologies) | SURFACTIF | 1 |
| F | OXYDE DE TITANE | COLORANT | 1,37 |
| | YELLOW 6 LAKE | COLORANT | 1,29 |
| | RED 7 | COLORANT | 0,3 |
| | BLUE 1 LAKE | COLORANT | 0,08 |
| | OXYDE DE FER NOIR | COLORANT | 0,16 |
| G | MICA-DIOXYDE DE TITANE-OXYDE DE FER BRUN | NACRE | 1 |
| | PARFUM | PARFUM | 0,05 |
| | Total | | 100% |

Protocole :

Dans un premier temps on disperse les pigments de la phase F dans la phase A et une partie de la phase B.

On mélange le reste des ingrédients liposolubles de la phase C et D et les cires de la phase E à une température de 100 °C puis on y ajoute le broyat et le reste de la phase B. On mélange le tout à une température de 100 °C jusqu'à l'obtention d'un mélange bien homogène.

Enfin on coule la composition dans un moule pour lui donner la forme d'un stick de diamètre 11.06 mm. On place ensuite le moule dans un congélateur à -18°C.

La dureté du stick à 20°C est de 117 $Nm^{-1}$

*Evaluation :*

A température ambiante (20°C), la composition ainsi obtenue lorsqu'elle est appliquée sur les lèvres, dépose très peu et est très accrocheuse (glisse mal sur les lèvres et se délite difficilement).

En revanche, après chauffage de l'extrémité du stick (en général en forme de biseau), à 60°C pendant 10 secondes, il est possible d'appliquer facilement (avec un bon glissant et un délitage aisé) le stick sur les lèvres et d'obtenir un dépôt de maquillage épais.

De plus, le maquillage déposé sur les lèvres avec la composition 2 préalablement chauffée présente une tenue de la couleur améliorée, ainsi qu'une brillance améliorée par rapport au dépôt de maquillage obtenu avec la même composition 2 non chauffée appliquée à température ambiante (20°C).

On observe également que le dépôt de maquillage réalisé avec la composition 2 préalablement chauffée présente, comparativement à celui réalisé avec la composition 1 préalablement chauffée, une tenue de la couleur améliorée, ainsi qu'une brillance améliorée immédiatement après l'application.

## Revendications

1. Procédé de maquillage et/ou de soin non thérapeutique des matières kératiniques humaines non fibreuses, notamment la peau, ses muqueuses ou les ongles, comportant les étapes consistant à :

   - amener au contact ou au voisinage d'un dispositif de chauffage une surface extérieure d'un morceau de composition cosmétique solide de manière à chauffer ledit morceau de façon localisée en vue de ne ramollir essentiellement que ladite surface extérieure et à en abaisser le coefficient de frottement dynamique et
   - appliquer ensuite la surface extérieure de la composition ainsi réchauffée sur la région à traiter,

ladite composition cosmétique solide comprenant dans un milieu physiologiquement acceptable :

(i) au moins un polyester susceptible d'être obtenu par réaction :

- d'un tétraol ayant de 4 à 10 atomes de carbone ;
- d'un acide saturé monocarboxylique, linéaire ou ramifié, ayant de 9 à 23 atomes de carbone ;
- d'un diacide carboxylique cyclique ayant de 6 à 12 atomes de carbone ; et
- d'un acide monocarboxylique aromatique ayant de 7 à 11 atomes de carbone,

(ii) et au moins un corps gras solide, choisi parmi les cires et les corps gras pâteux, et leur mélange.

**2.** Procédé selon la revendication précédente, dans lequel le(s)dit(s) polyester(s) est/sont susceptible(s) d'être obtenu (s) par réaction :

- de 10 à 30 % en poids d'un tétraol ayant de 4 à 10 atomes de carbone, en particulier choisi parmi le diglycérol et le pentaérythritol;
- de 40 à 80 % en poids d'un acide saturé monocarboxylique, linéaire ou ramifié, ayant de 9 à 23 atomes de carbone, en particulier choisi parmi l'acide stéarique et l'acide isostéarique;
- de 5 à 30 % en poids d'un diacide carboxylique cyclique ayant de 6 à 12 atomes de carbone, en particulier d'un acide isophtalique,
- de 0,1 à 10 % en poids d'un acide monocarboxylique aromatique ayant de 7 à 11 atomes de carbone, en particulier choisi parmi l'acide benzoïque et l'acide 4-tert-butyl benzoïque, et

les teneurs étant exprimées en pourcentage en poids par rapport au poids total du polyester.

**3.** Procédé selon l'une quelconque des revendications précédentes, dans lequel le(s)dit(s) polyester(s) est/sont choisi (s) parmi les polyesters d'acide benzoïque/acide isophtalique/acide isostéarique/pentaérythritol, les polyesters d'acide benzoïque/acide isophtalique/acide stéarique/pentaérythritol, et leurs mélanges

**4.** Procédé selon l'une quelconque des revendications précédentes, dans lequel ladite composition comprend au moins deux polyesters distincts l'un de l'autre.

**5.** Composition selon l'une quelconque des revendications précédentes, ladite composition comprenant de 1 à 60 % en poids de polyester, en particulier de 5 à 50 % en poids, et plus particulièrement de 10 à 45 % en poids, voire de 10 à 35 % en poids, et mieux de 15 à 30 % en poids de polyester par rapport au poids total de la composition.

**6.** Procédé selon l'une quelconque des revendications précédentes, dans lequel la composition comprend au moins une huile non volatile.

**7.** Procédé selon l'une quelconque des revendications précédentes, dans lequel la composition comprend au moins une huile de poids moléculaire supérieur à 500 g/mol.

**8.** Procédé selon la revendication précédente, dans lequel ladite huile est une huile siliconée phénylée.

**9.** Procédé selon l'une quelconque des revendications 6 à 8, dans lequel ladite huile est présente en une teneur allant de 1 à 90 % en poids, de préférence de 5 à 80 % en poids, mieux de 10 à 75 % en poids, mieux encore de 20 à 70 % en poids, et encore mieux de 30 à 70% en poids, par rapport au poids total de la composition.

**10.** Procédé selon l'une quelconque des revendications précédentes, dans lequel la composition cosmétique solide présente un coefficient de frottement dynamique sensible à la température, supérieur ou égal à 0,5 à 25 °C, mieux supérieur ou égal à 0,6 à 25°C.

**11.** Procédé selon l'une quelconque des revendications précédentes, dans lequel la composition cosmétique solide présente une dureté supérieure ou égale à 80 $Nm^{-1}$ à 20 °C, de préférence supérieure ou égale 100 $Nm^{-1}$, mieux supérieure ou égale à 120 $Nm^{-1}$.

**12.** Procédé selon l'une quelconque des revendications précédentes, dans lequel ledit coefficient de frottement dynamique est, à la température à laquelle la composition est chauffée, inférieur ou égal à 0,45, mieux à 0,4.

**13.** Procédé selon l'une quelconque des revendications précédentes, dans lequel la composition est sous forme de bâtonnet, notamment de diamètre supérieur ou égal à 7 mm.

**14.** Procédé selon l'une quelconque des revendications précédentes, dans lequel le procédé est un procédé de maquillage et par exemple la composition est un rouge à lèvres.

**15.** Kit comportant :

- une composition telle que définie dans l'une quelconque des revendications précédentes, et
- un dispositif de chauffage permettant de chauffer localement une surface d'un morceau de ladite composition.

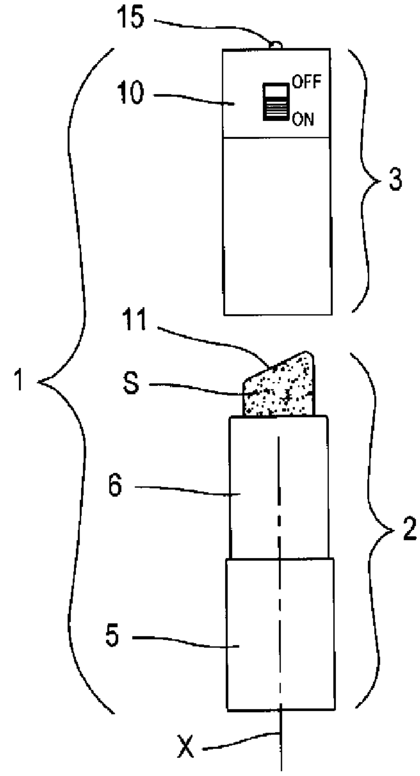


FIG. 1

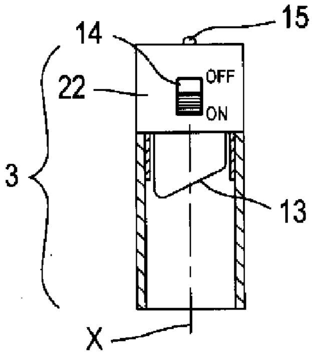


FIG. 2

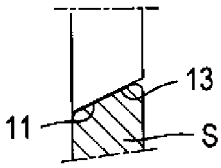


FIG. 3

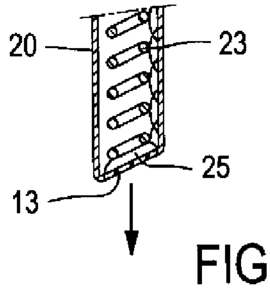


FIG. 4

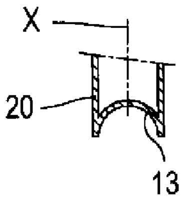


FIG. 5

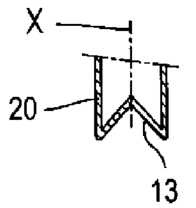


FIG. 6

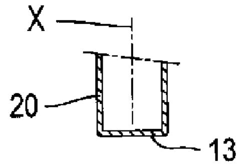


FIG. 7

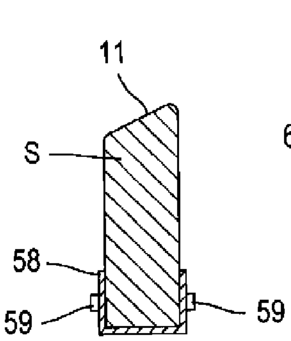


FIG. 10

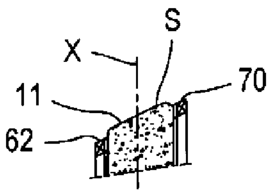


FIG. 12

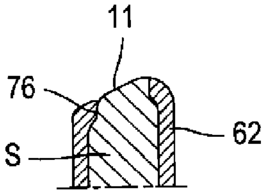


FIG. 13

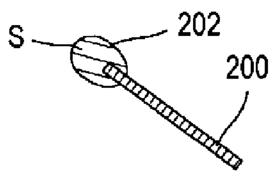


FIG. 14

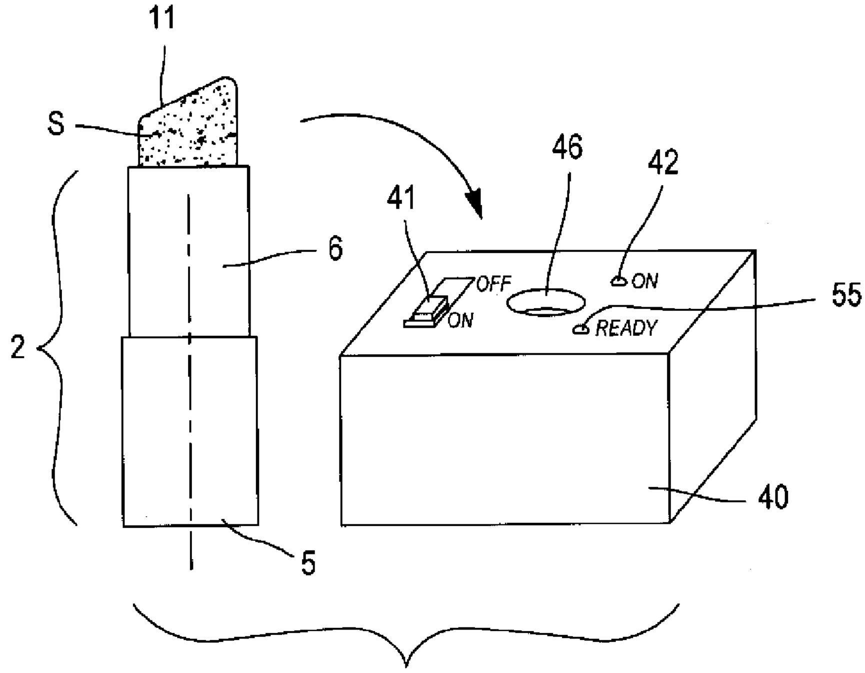


FIG. 8

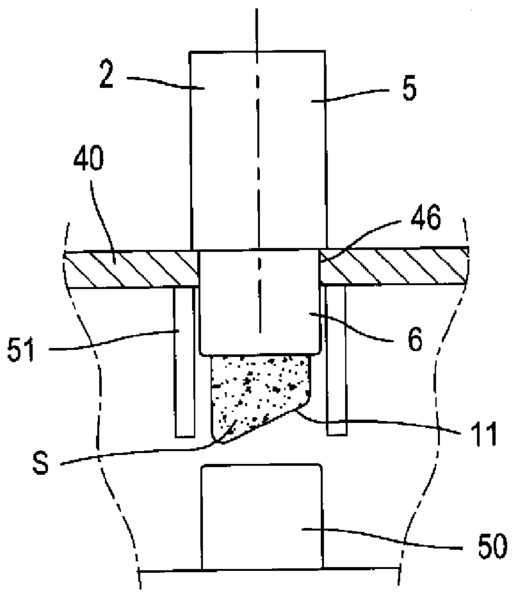


FIG. 9

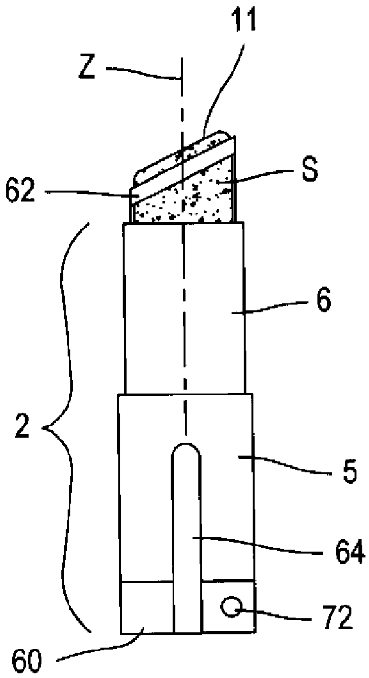


FIG. 11

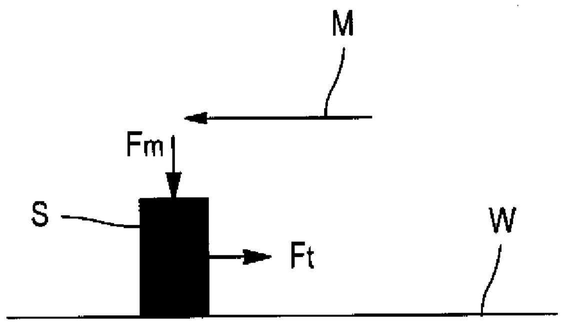


FIG. 15

Europäisches Patentamt
European Patent Office
Office européen des brevets

# RAPPORT DE RECHERCHE EUROPEENNE

Numéro de la demande
EP 11 15 0357

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2009/104133 A2 (OREAL [FR]; BLIN XAVIER [FR]; ILEKTI PHILIPPE [FR]; AUGUSTE FREDERIC [) 27 août 2009 (2009-08-27)<br>* le document en entier * | 1,15 | INV.<br>A61K8/02<br>A61K8/37<br>A61K8/85<br>A61K8/92<br>A61Q1/06<br>A45D40/08 |
| Y | US 2007/286831 A1 (KAMADA KENJI [JP] ET AL) 13 décembre 2007 (2007-12-13)<br>* alinéas [0005], [0006]; revendication 1 * | 1,15 | |
| Y | FR 2 921 829 A1 (OREAL [FR])<br>10 avril 2009 (2009-04-10)<br>* le document en entier * | 1,15 | |
| Y | FR 2 917 616 A1 (OREAL [FR])<br>26 décembre 2008 (2008-12-26)<br>* le document en entier * | 1,15 | |
| Y | EP 1 870 082 A2 (OREAL [FR])<br>26 décembre 2007 (2007-12-26)<br>* le document en entier * | 1,15 | DOMAINES TECHNIQUES RECHERCHES (IPC)<br>A61K<br>A61Q<br>A45D |

Le présent rapport a été établi pour toutes les revendications

| Lieu de la recherche | Date d'achèvement de la recherche | Examinateur |
|---|---|---|
| Munich | 11 mars 2011 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

1

EPO FORM 1503 03.82 (P04C02)

## ANNEXE AU RAPPORT DE RECHERCHE EUROPEENNE RELATIF A LA DEMANDE DE BREVET EUROPEEN NO.

EP 11 15 0357

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-03-2011

| Document brevet cité au rapport de recherche | Date de publication | Membre(s) de la famille de brevet(s) | Date de publication |
|---|---|---|---|
| WO 2009104133 A2 | 27-08-2009 | EP 2259697 A2 | 15-12-2010 |
| | | FR 2927782 A1 | 28-08-2009 |
| US 2007286831 A1 | 13-12-2007 | AUCUN | |
| FR 2921829 A1 | 10-04-2009 | WO 2009053587 A2 | 30-04-2009 |
| FR 2917616 A1 | 26-12-2008 | AUCUN | |
| EP 1870082 A2 | 26-12-2007 | BR PI0702122 A | 25-03-2008 |
| | | CN 101095654 A | 02-01-2008 |
| | | CN 101505715 A | 12-08-2009 |
| | | EP 2037875 A2 | 25-03-2009 |
| | | FR 2902653 A1 | 28-12-2007 |
| | | WO 2007148023 A2 | 27-12-2007 |
| | | WO 2007148024 A2 | 27-12-2007 |
| | | WO 2007148026 A2 | 27-12-2007 |
| | | JP 2008007504 A | 17-01-2008 |
| | | JP 2009541280 T | 26-11-2009 |
| | | KR 20070121586 A | 27-12-2007 |
| | | KR 20090053761 A | 27-05-2009 |
| | | KR 20090030319 A | 24-03-2009 |
| | | RU 2009101947 A | 27-07-2010 |
| | | US 2009202462 A1 | 13-08-2009 |
| | | US 2008152607 A1 | 26-06-2008 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2917616
- EP 1870082 A
- FR 2792190 A
- EP 0955039 A
- EP 708114 A
- US 2002005562 A
- US 5221534 A
- EP 0951897 A
- US 5156911 A
- WO 0119333 A
- US 5736125 A
- US 5519063 A
- EP 550745 A
- EP 0550745 A
- US 20070031361 A
- EP 571882 A
- EP 890583 A
- US 5998547 A
- WO 04055081 A
- FR 2232303 A
- WO 02056847 A
- WO 0247619 A
- US 5783657 A
- EP 1266647 A
- FR 0216039
- EP 1400234 A
- US 5874069 A
- US 5919441 A
- US 6051216 A
- US 5981680 A
- WO 2003106614 A
- US 3645705 A
- US 3148125 A
- US 5500209 A
- US 5998570 A
- EP 542669 A
- EP 787730 A
- EP 787731 A
- WO 9608537 A
- US 6340258 B
- US 6086276 A
- US 6371673 B
- US 5171096 A
- US 7293926 B
- US 20070286665 A1


### Littérature non-brevet citée dans la description


- **S. NOJIMA.** Melting behavior of poly(-caprolactone)-block-polybutadiène copolymers. *Macromolécules,* 1999, vol. 32, 3727-3734
- **B. BOUTEVIN et al.** Study of morphological and mechanical properties of PP/PBT. *Polymer Bulletin,* 1995, vol. 34, 117-123
- **P. RANGARAJAN et al.** Morphology of semi-crystalline block copolymers of ethylene-(ethylene-alt-propylene. *Macromolecules,* 1993, vol. 26, 4640-4645
- **P. RICHTER et al.** Polymer agregates with crystalline cores : the system poly(ethylene)-poly(ethylene-propylene. *Macromolécules,* 1997, vol. 30, 1053-1068
- **I.W. HAMLEY.** Cristallization in block copolymers. *Advances in Polymer Science,* 1999, vol. 148, 113-137